(19)

Europäisches
Patentamt

European
Patent Office

Office européen
des brevets

(11) **EP 4 682 890 A2**

(12) **EUROPEAN PATENT APPLICATION**

(43) Date of publication:
**21.01.2026  Bulletin 2026/04**

(21) Application number: **25221495.2**

(22) Date of filing: **23.02.2022**

(51) International Patent Classification (IPC):
***G16B 40/20*** (2019.01)

(52) Cooperative Patent Classification (CPC):
**G16B 30/00; G16B 40/20;** G16B 15/10

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **24.02.2021   US 202117184505**

(62) Document number(s) of the earlier application(s) in
accordance with Art. 76 EPC:
**22158108.5 / 4 030 438**

(71) Applicant: **Baidu USA LLC
Sunnyvale, California 94089 (US)**

(72) Inventors:
• **ZHANG, He
Sunnyvale, 94089 (US)**
• **ZHANG, Liang
Sunnyvale, 94089 (US)**

• **LI, Ziyu
Sunnyvale, 94089 (US)**
• **LIU, Kaibo
Sunnyvale, 94089 (US)**
• **LIU, Boxiang
Sunnyvale, 94089 (US)**
• **HUANG, Liang
Sunnyvale, 94089 (US)**

(74) Representative: **Maiwald GmbH
Elisenhof
Elisenstraße 3
80335 München (DE)**

Remarks:
This application was filed on 08.12.2025 as a
divisional application to the application mentioned
under INID code 62.

(54) **SYSTEMS AND METHODS FOR SEQUENCE DESIGN**

(57)   A messenger RNA (mRNA) vaccine has emerged as a promising direction to combat the COVID-19 pandemic. This requires an mRNA sequence that is stable and highly productive in protein expression, features to benefit from greater mRNA secondary structure folding stability and optimal codon usage. Sequence design remains challenging due to the exponentially many synonymous mRNA sequences encoding the same protein. The present disclosure presents embodiments of a linear-time approximation (LinearDesign) reducing the design to an intersection between a Stochastic Context Free Grammar (SCFG) and a Deterministic Finite Automaton (DFA). Embodiments of the LinearDesign may implement an mRNA sequence design using much reduced time with very limited loss. Various methodologies, e.g., finding alternative sequences based on k-best parsing or directly incorporating codon optimality, are presented for incorporating the codon optimality into the design. Embodiments of the LinearDesign may provide efficient computational tools to speed up and improve mRNA vaccine development.

**EP 4 682 890 A2**

**Description**

**TECHNICAL FIELD**

The present disclosure relates generally to systems and methods for sequence design. More particularly, the present disclosure relates to systems and methods for sequence design that can provide improved features, efficiency or uses.

**BACKGROUND**

The 2019 novel Coronavirus (SARS-CoV-2) caused an outbreak of viral pneumonia since late 2019 and has become a global pandemic. A messenger ribonucleic acid (mRNA) vaccine has emerged as a promising approach thanks to its rapid and scalable production and non-infectious and non-integrating properties. However, designing an mRNA sequence to achieve high stability and protein production remains a challenging problem. Recently, it is discovered that greater secondary structure folding stability and optimal codon usage synergize to increase protein expression. The design problem can therefore be formulated as finding the mRNA sequence(s) that are good in both secondary structure stability and codon optimality among the exponentially many synonymous sequences that encode the same protein.

Each amino acid may be translated by a codon, which has 3 adjacent mRNA nucleotides. For example, the start codon AUG translates into methionine, the first amino acid in any protein sequence. But due to redundancies in the genetic code ($4^3 = 64$ triplet codons for 21 amino acids), most amino acids may be translated from multiple codons. This fact makes the search space of mRNA design increase exponentially with protein length. For example, the spike protein of SARS-CoV-2 (the virus that causes the COVID-19 pandemic) contains 1,273 amino acids (plus the stop codon which is part of the mRNA but not part of a protein). There are about $2.4 \times 10^{632}$ mRNA candidates. The mRNA design problem, therefore, aims to exploit the redundancies in the genetic code to find more stable and productive mRNA sequences than the wild type in nature.

Accordingly, what is needed are systems and methods for sequence design that can provide improved features, efficiency or uses to address the challenges.

**SUMMARY**

**In** an aspect, there is provided a computer-implemented method for sequence design, including:

receiving a source sequence comprising multiple source sequence units;
building a unit deterministic finite automaton (DFA) for each of the multiple source sequence units, each unit DFA comprises multiple nodes including a start node and an end node, each DFA has one or more paths between the start node and the end node, each path comprises multiple edges with each edge coupled between two adjacent nodes;
concatenating at least unit DFAs for the multiple source sequence units into a single DFA representing candidate target sequences that translate into the source sequence;
intersecting a context free grammar (CFG) on the single DFA for an intersected CFG;
defining each nonterminal and start symbol in the intersected CFG;
defining one or more rules in the intersected CFG; and
searching in the intersected CFG for a desired target sequence having a sequence structure that minimizes an objective function.

In another aspect, there is provided another computer-implemented method for sequence design, including:

receiving a source sequence comprising multiple source sequence units;
building a unit deterministic finite automaton (DFA) for each of the multiple source sequence units, each unit DFA comprises multiple nodes including a start node and an end node, each DFA has one or more paths between the start node and the end node, each path comprises multiple edges with each edge coupled between two adjacent nodes;
concatenating at least unit DFAs for the multiple source sequence units into a single DFA representing candidate target sequences that translate into the source sequence, each target sequence comprising multiple target sequence units respectively translating into the multiple source sequence units;
intersecting a context free grammar (CFG) on the single DFA for an intersected CFG;
searching in the intersected CFG for a desired target sequence having a sequence structure that minimizes an objective function.,

In another aspect, there is provided a non-transitory computer-readable medium or media comprising one or more sequences of instructions which, when executed by at least one processor, causes steps for sequence design described in the above aspects are implemented.

With the disclosure, systems and methods for sequence design that can provide improved features, efficiency or uses are provided.

## BRIEF DESCRIPTION OF THE DRAWINGS

References will be made to embodiments of the disclosure, examples of which may be illustrated in the accompanying figures. These figures are intended to be illustrative, not limiting. Although the disclosure is generally described in the context of these embodiments, it should be understood that it is not intended to limit the scope of the disclosure to these particular embodiments. Items in the figures may not be to scale.

Figure ("FIG.") 1 depicts exemplary Deterministic Finite Automatons (DFAs) representing 4 different types of amino acids and their codons, according to embodiments of the present disclosure.

FIG. 2 depicts a single DFA of a protein sequence "methionine leucine", according to embodiments of the present disclosure.

FIG. 3 depicts a process for establishing DFA representation for source and target sequence search space, according to embodiments of the present disclosure.

FIG. 4 depicts one of the best derivations of the intersected grammar, demonstrating the path through the SCFG and the DFA, according to embodiments of the present disclosure.

FIG. 5 depicts the best derivation of a sequence CCAAAGG by the Nussinov-Jacobson grammar, according to embodiments of the present disclosure.

FIG. 6 depicts a process for CFG intersection with DFA, according to embodiments of the present disclosure.

FIG. 7 depicts a process for a bottom-up dynamic methodology for mRNA design, according to embodiments of the present disclosure.

FIG. 8 depicts a pseudocode for the bottom-up dynamic methodology for mRNA design, according to embodiments of the present disclosure.

FIG. 9 depicts an exemplary pseudocode for Update function in a bottom-up dynamic methodology, according to embodiments of the present disclosure.

FIG. 10 depicts an exemplary pseudocode for Backtrace function in a bottom-up dynamic methodology, according to embodiments of the present disclosure.

FIG. 11 depicts a process using a left-to-right dynamic methodology for mRNA design, according to embodiments of the present disclosure.

FIG. 12 gives the pseudocode of simplified LinearDesign algorithm for the Nussinov model based on left-to-right dynamic programming and beam pruning, according to embodiments of the present disclosure.

FIG. 13 depicts exemplary pseudocodes for Backtrace function in a left-to-right dynamic methodology, according to embodiments of the present disclosure.

FIG. 14 depicts exemplary pseudocodes for Beamprune function in a left-to-right dynamic methodology, according to embodiments of the present disclosure.

FIG. 15 depicts a DFA graph of "serine" with Codon Adaptation Index (CAI) as edge weight different at the last edges, according to embodiments of the present disclosure.

FIG. 16 depicts a DFA graph of "serine" with CAI as edge weight different before the last edges, according to embodiments of the present disclosure.

FIG. 17 depicts a process of shifting edge weight difference for edges in a DFA, according to embodiments of the present disclosure.

FIG. 18 depicts runtime comparison between CDSfold and LinearDesign, according to embodiments of the present disclosure.

FIG. 19A depicts free energy gap of LinearDesign with beam size $b = 1,000$ compared with exact search, according to embodiments of the present disclosure.

FIG. 19B depicts percentage of LinearDesign free energy gap changes linearly with mRNA sequence length, according to embodiments of the present disclosure.

FIG. 19C depicts percentage of LinearDesign free energy gap changes with beam size, according to embodiments of the present disclosure.

FIG. 20 depicts two-dimensional comparisons (MFE and CAI) between wildtype mRNA sequence, random sequences, and designed sequences on the spike protein of SARS-CoV-2, according to embodiments of the present disclosure.

FIG. 21 depicts secondary structures of the wildtype and designed mRNA sequences that translate into the spike protein of SARS-CoV-2, according to embodiments of the present disclosure.

FIG. 22 depicts a simplified block diagram of a computing device/information handling system, according to embodiments of the present disclosure.

## DETAILED DESCRIPTION OF EMBODIMENTS

In the following description, for purposes of explanation, specific details are set forth in order to provide an understanding of the disclosure. It will be apparent, however, to one skilled in the art that the disclosure can be practiced without these details. Furthermore, one skilled in the art will recognize that embodiments of the present disclosure, described below, may be implemented in a variety of ways, such as a process, an apparatus, a system, a device, or a method on a tangible computer-readable medium.

Components, or modules, shown in diagrams are illustrative of exemplary embodiments of the disclosure and are meant to avoid obscuring the disclosure. It shall also be understood that throughout this discussion that components may be described as separate functional units, which may comprise sub-units, but those skilled in the art will recognize that various components, or portions thereof, may be divided into separate components or may be integrated together, including, for example, being in a single system or component. It should be noted that functions or operations discussed herein may be implemented as components. Components may be implemented in software, hardware, or a combination thereof.

Furthermore, connections between components or systems within the figures are not intended to be limited to direct connections. Rather, data between these components may be modified, re-formatted, or otherwise changed by intermediary components. Also, additional or fewer connections may be used. It shall also be noted that the terms "coupled," "connected," "communicatively coupled," "interfacing," "interface," or any of their derivatives shall be understood to include direct connections, indirect connections through one or more intermediary devices, and wireless connections. It shall also be noted that any communication, such as a signal, response, reply, acknowledgement, message, query, etc., may comprise one or more exchanges of information.

Reference in the specification to "one or more embodiments," "preferred embodiment," "an embodiment," "embodiments," or the like means that a particular feature, structure, characteristic, or function described in connection with the embodiment is included in at least one embodiment of the disclosure and may be in more than one embodiment. Also, the appearances of the above-noted phrases in various places in the specification are not necessarily all referring to the same embodiment or embodiments.

The use of certain terms in various places in the specification is for illustration and should not be construed as limiting. A service, function, or resource is not limited to a single service, function, or resource; usage of these terms may refer to a grouping of related services, functions, or resources, which may be distributed or aggregated. The terms "include," "including," "comprise," and "comprising" shall be understood to be open terms and any lists the follow are examples and not meant to be limited to the listed items. A "layer" may comprise one or more operations. The words "optimal," "optimize," "optimization," and the like refer to an improvement of an outcome or a process and do not require that the specified outcome or process has achieved an "optimal" or peak state. The use of memory, database, information base, data store, tables, hardware, cache, and the like may be used herein to refer to system component or components into which information may be entered or otherwise recorded.

In one or more embodiments, a stop condition may include: (1) a set number of iterations have been performed; (2) an amount of processing time has been reached; (3) convergence (e.g., the difference between consecutive iterations is less than a first threshold value); (4) divergence (e.g., the performance deteriorates); and (5) an acceptable outcome has been reached.

One skilled in the art shall recognize that: (1) certain steps may optionally be performed; (2) steps may not be limited to the specific order set forth herein; (3) certain steps may be performed in different orders; and (4) certain steps may be done concurrently.

Any headings used herein are for organizational purposes only and shall not be used to limit the scope of the description or the claims. Each reference/document mentioned in this patent document is incorporated by reference herein in its entirety.

It shall be noted that any experiments and results provided herein are provided by way of illustration and were performed under specific conditions using a specific embodiment or embodiments; accordingly, neither these experiments nor their results shall be used to limit the scope of the disclosure of the current patent document.

It shall also be noted that although embodiments described herein may be within the context of SARS-CoV-2 Genome, aspects of the present disclosure are not so limited. Accordingly, the aspects of the present disclosure may be applied or adapted for use in other genetic material, or non-genetic material, such as antibody design.

### A. General Introduction

The 2019 novel Coronavirus (SARS-CoV-2) caused an outbreak of viral pneumonia since late 2019 and has become the global COVID-19 pandemic. A messenger RNA (mRNA) vaccine has emerged as a promising approach thanks to its rapid and scalable production and non-infectious and non-integrating properties. However, designing an mRNA sequence to achieve high stability and protein production remains a challenging problem. Recently, it is discovered that greater secondary structure folding stability and optimal codon usage synergize to increase protein expression. The design problem can therefore be formulated as finding the mRNA sequence(s) that are good in both secondary structure stability and codon optimality among the exponentially many synonymous sequences that encode the same protein.

Each amino acid may be translated by a codon, which has 3 adjacent mRNA nucleotides. For example, the start codon AUG translates into methionine, the first amino acid in any protein sequence. But due to redundancies in the genetic code ($4^3 = 64$ triplet codons for 21 amino acids), most amino acids may be translated from multiple codons. This fact makes the search space of mRNA design increase exponentially with protein length. For example, the spike protein (GenBank QHD43416.1) of SARS-CoV-2 (the virus that causes the COVID-19 pandemic) contains 1,273 amino acids (plus the stop codon which is part of the mRNA but not part of a protein). There are about $2.4 \times 10^{632}$ mRNA candidates. The mRNA design problem, therefore, aims to exploit the redundancies in the genetic code to find more stable and productive mRNA sequences than the wild type in nature.

The present patent disclosure shows that this design problem may be reduced to a classical notion in formal language theory and computational linguistics, namely the intersection between a Stochastic Context Free Grammar (SCFG) and a Deterministic Finite Automaton (DFA). Here the SCFG represents the folding free energy model and the DFA represents the set of all possible synonymous mRNA sequences that code a given protein. Novelty of the present patent disclosure includes the use of DFA to encode the mRNA search space and solving the design problem via the intersection of SCFG and DFA. This intersection may be done in $O(n^3)$ time, where $n$ is the mRNA sequence length, but it could still be too slow for large $n$ (e.g., n = 3 × (1273 + 1) = 3, 822 nucleotides for the spike protein of SARS-CoV-2). The present disclosure discloses embodiments of a linear-time approximate model (also referred as "LinearDesign" hereinafter) to reduce runtime. The present disclosure further discloses various methodologies for incorporating the codon optimality into the design, including one methodology based on k-best parsing to find alternative sequences and one methodology directly incorporating codon optimality into the dynamic programming.

Some prior efforts used dynamic programming, including CDSfold (Goro Terai, et al., CDSfold: an algorithm for designing a proteincoding sequence with the most stable secondary structure. Bioinformatics, 32(6):828-834, 2016, which is incorporated by reference herein in its entirety), for most stable RNA design. Multiple aspects of differences exist between the present disclosure and those prior efforts. First, in the present disclosure, the sequence design problem is reduced to "CFG-DFA intersection", a classical problem in formal language theory and computational linguistics, which is very general and may be applied to other scenarios with alternative inputs. While algorithms adopted in prior efforts were ad hoc. Second, codon optimality is integrated in the optimization in the present disclosure. Third, embodiments of a linear-time approximate version with greatly reduced runtime for long sequences with small or no sacrifices in search quality are disclosed in the present disclosure.

## B. Embodiments for LinearDesign Methodology

In one or more embodiments of the present disclosure, the sequence design problem is formulated as follows: given a source sequence (e.g., a protein sequence) $p = p_1 \ldots p_m$ where each $p_i$ is a unit (e.g., an amino acid) of the source sequence, searching, among all candidate target sequences (e.g., mRNA sequences) r that translate into the source sequence, the best target sequence r*(p), defined as the sequence minimizing an objective function (e.g., folding free energy change):

$$\mathbf{r}^*(\mathbf{p}) = \underset{\mathbf{r} \in \mathbf{RNA(p)}}{\operatorname{argmin}} \mathbf{MFE(r)} \qquad (1)$$

$$\mathbf{MFE(r)} = \underset{\mathbf{s} \in \mathrm{structure(r)}}{\min} \Delta G^o(\mathbf{r, s}) \qquad (2)$$

where MFE refers to minimum free energy, **RNA(p) = {r |** protein(r) = p} is the search space, structure(r) is the set of all possible secondary structure for RNA sequence r, and $\Delta G^o$(r, s) is the free energy change of structure s for RNA r according to an energy model. It shall be noted that an mRNA sequence length is n = 3($m$ + 1) due to the existence of a final stop codon, which is not translated into the protein sequence.

Disclosure hereinafter shows embodiments of how to represent the exponentially large search space **RNA(p)** compactly via DFAs, and embodiments of how to do this **argmin** search (over the product of two exponentially large spaces) efficiently via dynamic programming, which may be reduced to the CFG intersection with DFA.

**1.** Embodiments of DFA representation for source and target sequence search space

In the fields of formal language theory and computational linguistics, the DFA graph is typically used to encode ambiguities in languages. It is noticed that the ambiguity of target sequence unit (e.g., a codon) choice for a source sequence unit (e.g., an amino acid) may be similar as to the language ambiguity problem, and may be represented with a DFA graph. Described hereinafter are embodiments of representing source and target sequence units (e.g., amino acid codons) using DFAs.

In one or more embodiments, a DFA may be referred as a directed graph with labeled edges and distinct start and end nodes. In one or more embodiments, each edge may be labeled by a nucleotide, such that each start-to-end path represents a triplet codon. In one or more embodiments, the DFA is a 5-tuple $\langle Q, \Sigma, \delta, q_0, F \rangle$ where Q is the set of nodes, $\Sigma$ is the alphabet (e.g., $\Sigma$ = {A, C, G, U}, where *A, C, G, and U* are respectively referred as adenine, cytosine, guanine, and uracil, which are four bases for RNA), $q_0$ is the start node, F is the set of end nodes (in this work the end node is unique, i.e., | F| = 1), and $\delta$ is the transition function that takes a node *q* and a symbol $a \in \Sigma$ and returns the next node q', i.e., $\delta(q, a)$ = q' encodes a labeled edge $q \xrightarrow{a} q'$ .

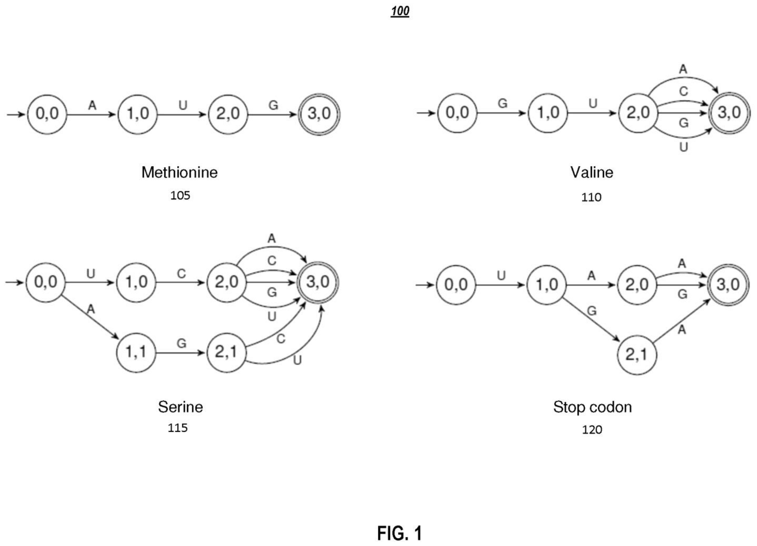

FIG. 1 depicts exemplary unit DFAs representing 4 different types of amino acids and their codons, according to embodiments of the present disclosure. All these unit DFAs have (0, 0) and (3, 0) as their start node and end node, respectively. The DFA representation 105 for methionine has only one path (AUG). Besides methionine, tryptophan is the only other amino acid with a single codon path in the standard genetic code. The unit DFA 110 for amino acid valine codon has multiple choices at the third nucleotide. Most amino acids (15 out of 21) are of this type, with 2-4 paths. The unit DFA 115 for serine has the most complex case with 6 paths and branching happens at the start node. Leucine and arginine also have similar DFAs, with 6 paths and branching happens at the start node. The unit DFA 120 for the stop codon is special because branching happens at the second step, at node (1, 0).

In one or more embodiments, after building unit DFAs for each amino acid, the DFAs are concatenated into a single DFA *D*(p) for a source sequence p (e.g., an amino acid sequence for a protein). The single DFA represents all candidate target sequences (e.g., mRNA sequences) that translate into the source sequence (e.g., the protein), which may be presented as:

$$D(\mathbf{p}) = D(p_1) \circ D(p_2) \circ \cdots \circ D(p_m) \circ D(STOP) \qquad (3)$$

In one or more embodiments, the single DFA D (p) may be formed by stitching the end node of each unit DFA with the start node of the next. For mRNA sequences, the new end node of the single DFA may be expressed as (3(*m* + 1), 0).

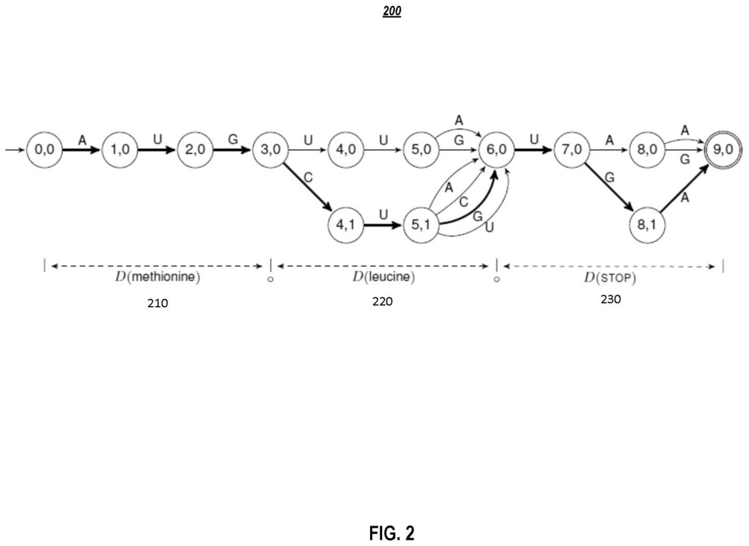

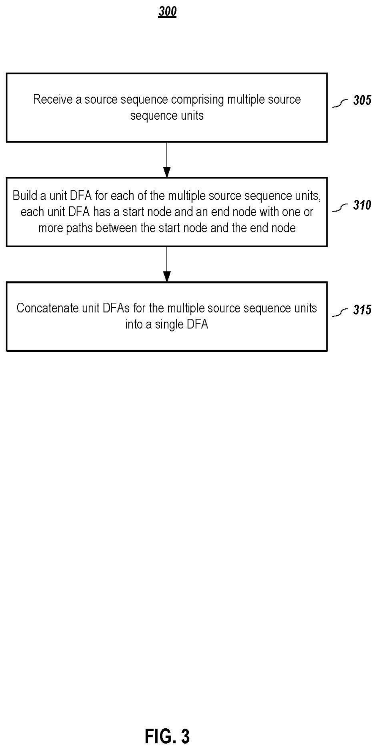

FIG. 2 depicts a single DFA of a protein sequence "methionine leucine", according to embodiments of the present disclosure. The single DFA comprise a unit DFA 210 for methionine, a unit DFA 220 for leucine, and a unit DFA 230 for the stop codon. As shown in FIG. 2, the single DFA may have multiple paths with each path having 9 nucleotides. The thick arrows indicate the best mRNA sequence (AUGCUGUGA, as shown in FIG. 3) after intersecting this DFA with the context-free grammar.

In one or more embodiments, out_edges(q) is defined to be set of outgoing edges from node *q*, and in_edges(*q*) is defined to be the set of incoming edges:

$$\text{out\_edges}(q) = \{(q', a) | \delta(q, a) = q'\} \qquad (4)$$

$$\text{in\_edges}(q) = \{(q', a) | \delta(q', a) = q\} \qquad (5)$$

For the DFA in FIG. 2, out_edges((3, 0)) = {((4, 0), U), ((4, 1), C)} and in_edges((9, 0)) = {((8, 0), A), ((8, 0), G), ((8, 1), A)}.

FIG. 3 depicts a process for establishing DFA representation for source and target sequence search space, according to embodiments of the present disclosure. In step 305, a source sequence comprising multiple source sequence units is received. The source sequence may be a protein sequence and the source sequence units are amino acids. In step 310, a unit DFA is built for each of the multiple source sequence units. Each unit DFA comprises multiple nodes including a start node and an end node. Each DFA has one or more paths between the start node and the end node with each path representing one target sequence that translates into the source sequence unit. Each path comprises multiple edges with each edge coupled between two different nodes. In one or more embodiments, each DFA comprises all possible codons that may translate into a corresponding amino acid. In step 315, unit DFAs for the multiple source sequence units are concatenated into a single DFA by stitching the end node of each unit DFA with the start node of the next unit.

**2. Embodiments of context-free grammar (CFG) intersection with DFA**

In formal language theory, a formal grammar is considered as a CFG when its production rules can be applied regardless of the context of a nonterminal. A stochastic context-free grammar (SCFG) is a CFG in which each rule is augmented with a weight. In one or more embodiments, an SCFG may be expressed as a 4-tuple $\langle N, \Sigma, P, S\rangle$ where $N$ is the set of non-terminals, $\Sigma$ is the set of terminals (identical to the alphabet in the DFA, which may be expressed as {A, C, G, U} for mRNA sequences), P is the set of weight-associated context-free writing rules, and S $\in N$ is the start symbol. Each rule in P has the form $A \xrightarrow{w} (N \cup \Sigma)^*$ where $A \in N$ is a nonterminal that may be rewritten according to this rule into a sequence of non-terminals and terminals (* means repeating zero or more times) and $w \in \mathbb{R}$ is the weight associated with this rule.

In one or more embodiments, SCFGs may be used to represent RNA folding, a process by which a linear RNA molecule acquires secondary structure through intra-molecular interactions. The weight of a derivation (parse tree, or a secondary structure in this case) may be a sum of weights of the productions used in that derivation.

In one or more embodiments, for a very simple Nussinov-Jacobson model (which simplifies the energy model to the number of base pairs), the following SCFG G may be defined as:

$$S \xrightarrow{0} S\,S \qquad\qquad (6)$$

$$S \xrightarrow{-1} A\,S\,U \mid U\,S\,A \mid C\,S\,G \mid G\,S\,C \mid G\,S\,U \mid U\,S\,G \qquad (7)$$

$$S \xrightarrow{0} N\,S \mid S\,N \mid N\,N\,N \qquad\qquad (8)$$

$$N \xrightarrow{0} A \mid C \mid G \mid U \qquad\qquad (9)$$

Here expression (6) is the bifurcating case; expression (7) is the base-pairing case (with weight -1, and the negative score mirrors the free energy minimization problem); expression (8) is the unpaired cases; and expression (9) is for terminal rule. In one or more embodiments, the unpaired expression $S \xrightarrow{0} N\,N\,N$ makes sure the minimum hairpin length is 3, i.e., no sharp turn is allowed. Hairpins are a common type of secondary structure in RNA molecules. A hairpin (also referred as a hairpin loop) is an unpaired loop of mRNA that is created when an mRNA strand folds and forms base pairs with another section of the same strand.

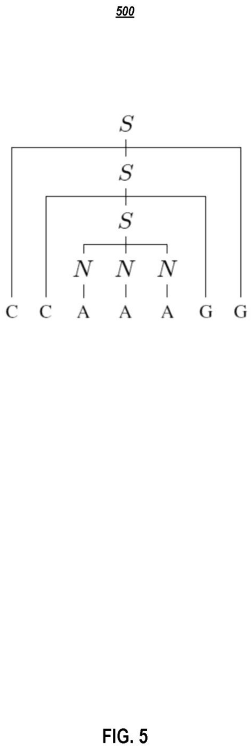

In one or more embodiments, the standard RNA secondary structure prediction problem under a Nussinov model may be casted as a parsing problem: given the above SCFG G and an input RNA sequence, what is the minimum-weight derivation in G that may generate sequence? For example, for an input CCAAAGG sequence, the best secondary structure derivation using the Nussinov- Jacobson grammar is showed in FIG. 5.

In one or more embodiments, the mRNA design problem may be an extension of the above single-sequence folding problem to the case of multiple inputs: instead of finding the minimum energy structure (minimum weight derivation) for a given sequence, the minimum energy structure (and its corresponding sequence) needs to be found among all possible structures for all candidate sequences. This may be solved by intersecting the SCFG G on the single (e.g., the protein) DFA $D$, which results in a bigger SCFG $(G' = G \cap D)$ and find the best derivation in $G'$.

In one or more embodiments, in the intersected grammar G', each nonterminal has a form $q_1 A q_2$, where $A \in N$ is an original nonterminal in G; $q_1$ and $q_2$ are two nodes in D; and the new start symbol is $q_0 S q_n$, where S is the original start symbol in G; $q_0$ and $q_n$ are the start and end nodes in D.

In one or more embodiments, the bifurcation rule $S \xrightarrow{0} S\,S$ may become:

$$q_1 S q_3 \xrightarrow{0} q_1 S q_2 \; q_2 S q_3 \qquad\qquad (10)$$

for all $(q_1, q_2, q_3)$ node triplets in $D$. It may be seen that this intersection construction generalizes a Cocke-Kasami-Younger (CKY) algorithm where the triple of states $(q_1, q_2, q_3)$ generalizes the triple of string indices $(i, k, j)$. The CKY algorithm is a special case of intersection when the DFA only encodes one string, e.g., when a protein is made of amino acids that have only one codon (methionion and tryptophan). In one or more embodiments, this intersection construction is used for parsing in a DFA to account for ambiguity in target sequence (e.g., mRNA codon) choice.

In one or more embodiments, the terminal rule $N \xrightarrow{0} A$ may become $q_1 N q_2 \xrightarrow{0} q_1 A q_2$ only if there is a labeled edge $q_1 \xrightarrow{A} q_2$ in D, i.e., $\delta(q_1, A) = q_2$.

In one or more embodiments, the intersected grammar G' will have $N|Q|^2$ nonterminals and $P|Q|^3$ rules in the worst case ($|Q|$ is the number of nodes in D). This resembles the space and time complexities of the CKY algorithm (where $|Q| = n$). Indeed, intersection is a generalization of CKY from fixed input (RNA folding) to lattice input (mRNA design).

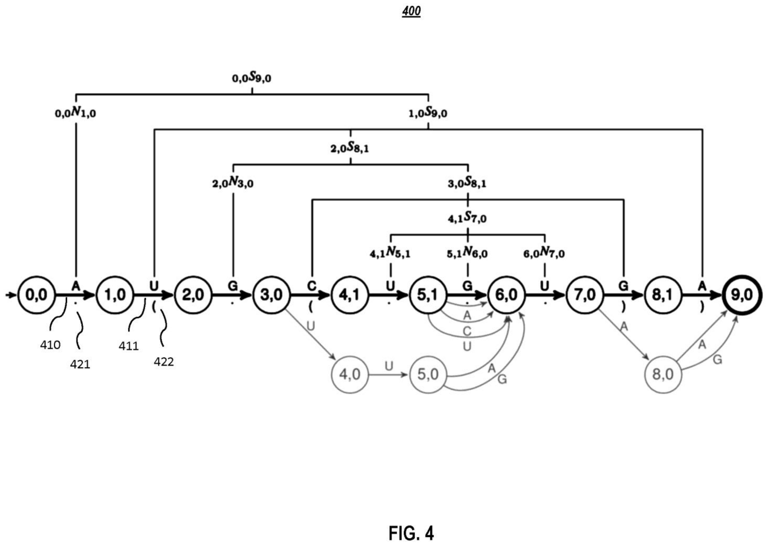

Now the next step is to find the best (minimum weight) derivation in G', from which the best mRNA sequence and its corresponding structure may be read off. FIG. 4 depicts one of the best derivations for the single DFA in Fig. 2, according to embodiments of the present disclosure. The derivation shown in thick black arrows (410, 411...) demonstrates the path through the SCFG and the DFA (there may be multiple best trees due to the simple energy model). The corresponding secondary structure is shown in dot-bracket format (421, 422...) below the solid arrows. The rest of the DFA are shown in light gray. Dot-bracket notation is a way of representing secondary structure of RNA. Each character represents a base. Open parentheses indicate that the base is paired to another base ahead of it. Closed parentheses indicate that a base is paired to another base behind it. Dots indicate an unpaired base. The number of open and closed parentheses will always be equal.

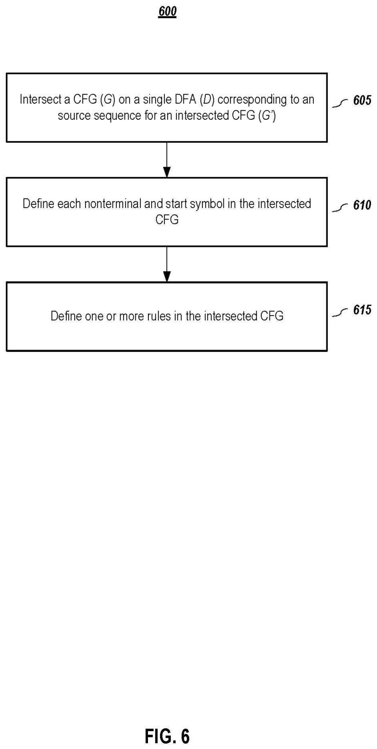

FIG. 6 depicts a process for CFG intersection with DFA, according to embodiments of the present disclosure. In step 605, a CFG (G) is intersected on a single DFA (D) corresponding to a source sequence (e.g., a protein sequence) for an intersected CFG $G'$ ($G' = G \cap D$). In one or more embodiments, the CFG is a SCFG. In step 610, each nonterminal in G' is defined as a form $(q_1 A q_2)$ comprising two nodes ($q_1$ and $q_2$) in D and an original nonterminal ($A \in N$) in G; the new nonterminal in G' is defined as a combination $(q_0 S q_n)$ comprising the start and end nodes ($q_0$ and $q_n$) in D and the original start $(S)$ symbol in G. In step 615, one or more rules, e.g., a bifurcation rule, a pairing rule, a termination rule, etc., in the intersected CFG G' are defined.

## 3. Embodiments of bottom-up dynamic methodology on Nussinov model

Described in this subsection are embodiments of dynamic methodology based on CFG intersection with DFA. A bottom-up dynamic programming on the Nussinov-Jacobson energy model is used first as an introduction.

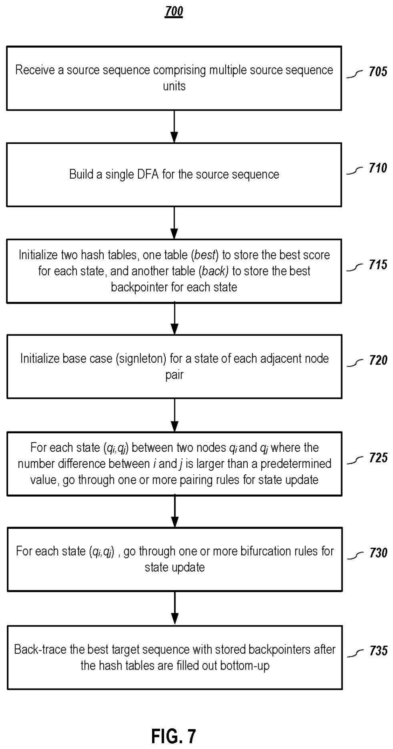

FIG. 7 depicts a process for a bottom-up dynamic methodology for mRNA design, according to embodiments of the present disclosure. A source sequence (e.g., a protein sequence) is received (705) and a single DFA for the source sequence is built (710) afterwards. The single DFA may be built according to the steps shown in FIG. 3. Two hash tables, *best* to store the best score for each state $(q_i, q_j)$, and *back* to store the best backpointer for each state $(q_i, q_j)$ between two different nodes $q_i$ and $q_j$, are initialized (715). The base case (singleton rule) for a state $(q_i, q_{i+1})$ for each adjacent node pair is initialized (720) with the best score for the state setting as zero initially and the best backpointer for the state setting as $nuc_i$ initially ($best[q_i, q_{i+1}] \leftarrow 0$ and $back[q_i, q_{i+1}] \leftarrow nuc_i$ for each state $(q_i, q_{i+1})$, where $nuc_i$ is the edge between $q_i$ and $q_{i+1}$). Next, each state $(q_i, q_j)$ between two nodes $q_i$ and $q_j$ where the difference between $j$ and $i$ is larger than a predetermined value, e.g., $j - i > 4$, goes through (725) one or more pairing rules and then goes through (730) one or more bifurcation rules for state updates if a better (lower) score is found. After filling out the hash tables bottom-up, the best target sequence (e.g., mRNA sequence) together with stored backpointers may be back traced (735). In one or more embodiments, the constraint of difference between $j$ and $i$ being larger than a predetermined value, e.g., $j - i > 4$, is required in RNA folding to ensure "no sharp turn".

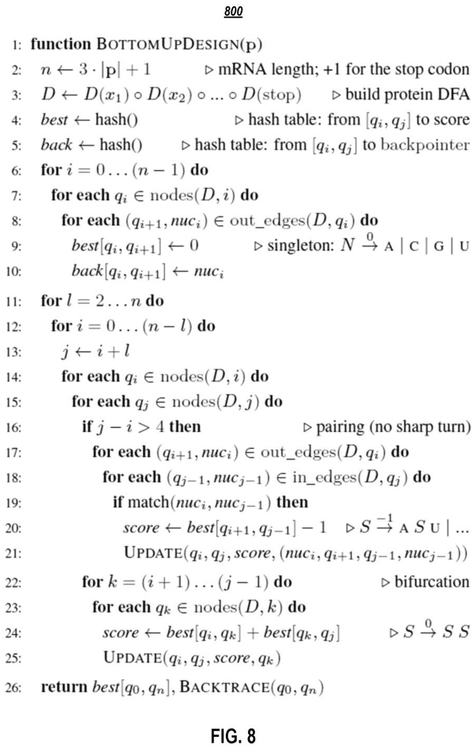

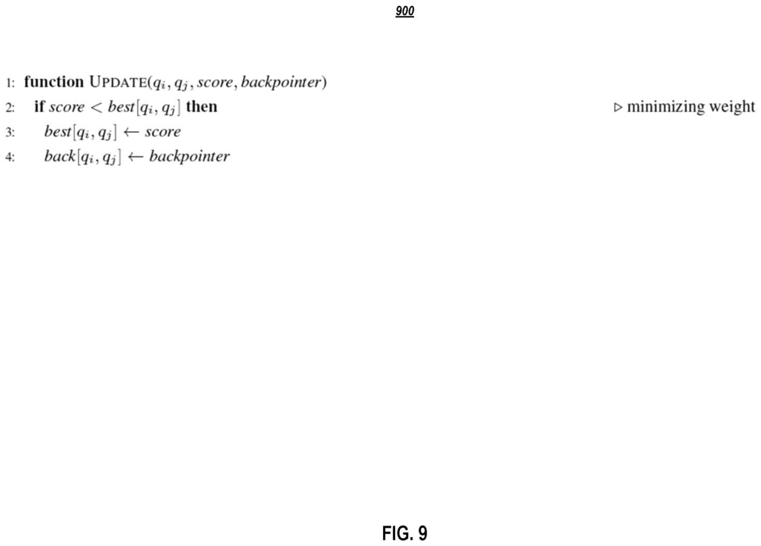

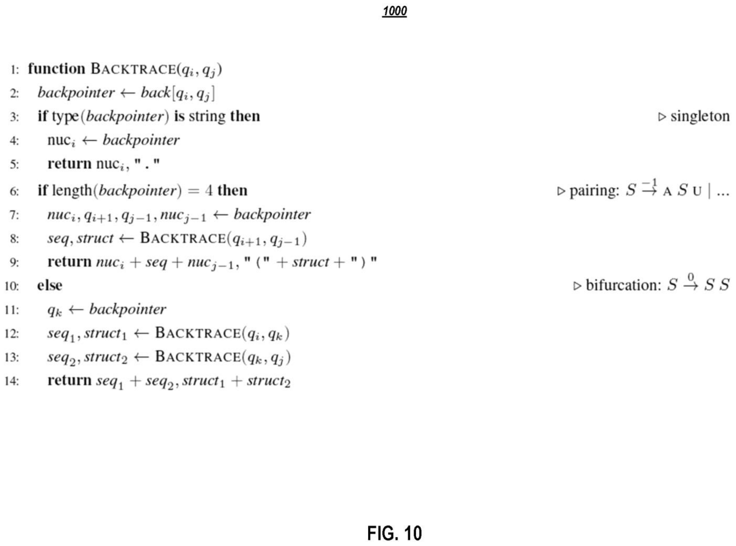

FIG. 8 gives an exemplary pseudocode for the bottom-up dynamic methodology, according to embodiments of the present disclosure. FIG. 9 and FIG. 10 respectively depict exemplary pseudocodes for Update function and Backtrace function in a bottom-up dynamic methodology, according to embodiments of the present disclosure. One shall understand that the pseudocode may be created using a computer language other than the language shown in FIGs.8-10.

## 4. Embodiments of left-to-right dynamic methodology with beam pruning

Algorithm based on bottom-up dynamic methodology runs in cubic time, and may be still slow for long sequences. The present patent document discloses embodiments of a linear-time approximation methodology, which may be used for various applications including mRNA design. In one or more embodiments, beam pruning may be applied to significantly narrow down the search space without sacrificing too much search quality.

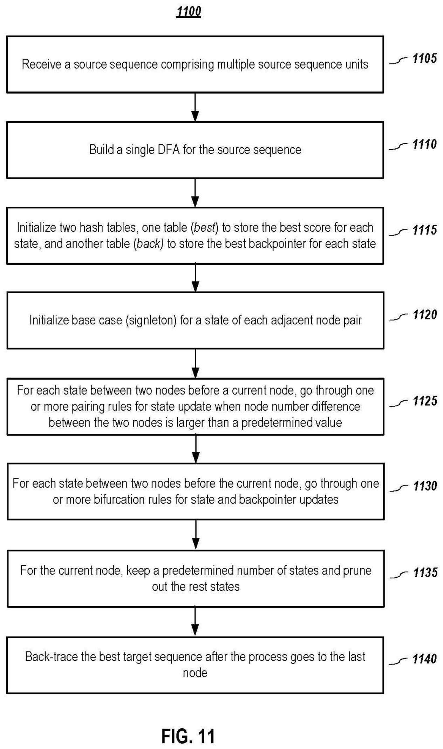

FIG. 11 depicts a LinearDesign process using a left-to-right dynamic methodology for mRNA design, according to embodiments of the present disclosure. Steps 1105-1120 may be similar to steps 705-720 for the bottom-up dynamic process shown in FIG. 7. LinearDesign replaces at least part of the bottom-up dynamic programming with a left-to-right parsing. Next, for a current position (e.g., the jth) of in the mRNA sequence, each state between any two nodes, which are before the current position, goes through (1125) one or more pairing rules (if the node number difference between the two nodes is larger than a predetermined value, e.g., 4) and then goes through (1130) one or more bifurcation rules for state and backpointer updates if a better score is found.

In one or more embodiments, for the current position (e.g., the $j$-th position of mRNA sequence), only a predetermined number of states (e.g., top $b$) with the lowest free energy are kept and the less promising states (or the rest states) are pruned out (1135), since they are unlikely to be the optimal sequence. Here $b$, the beam size, is a user-adjustable parameter to balance runtime and search quality. This approximation leads to a significant runtime reduction from $O(n^3)$ to $O(nb^2)$. In one or more embodiments, in LinearDesign, a beam size $b$ larger than 100 may usually be used because of the large search space.

After the process goes to the last node, the best target sequence (e.g., mRNA sequence) together with stored backpointers may be back traced (1140).

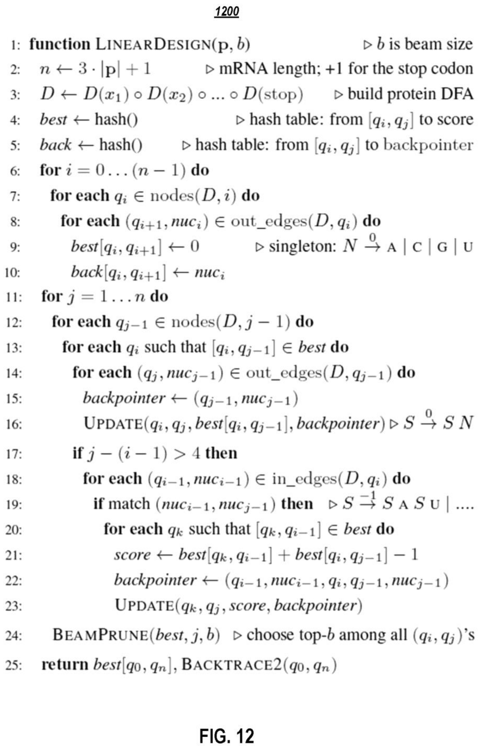

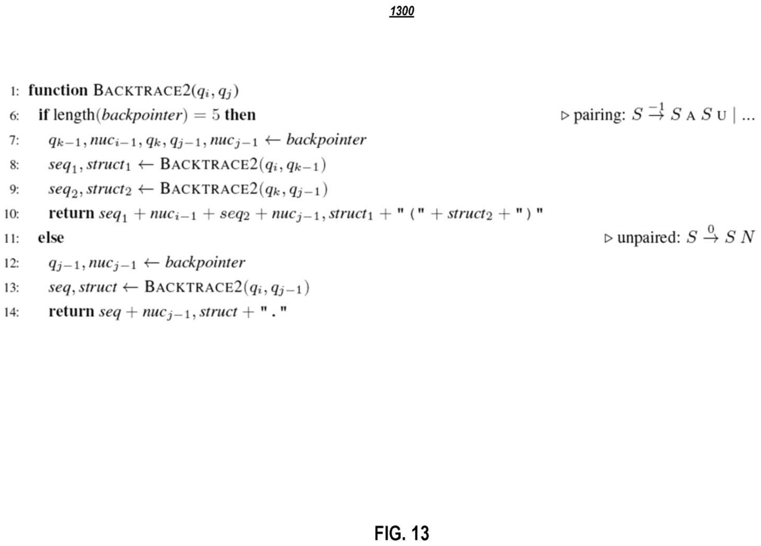

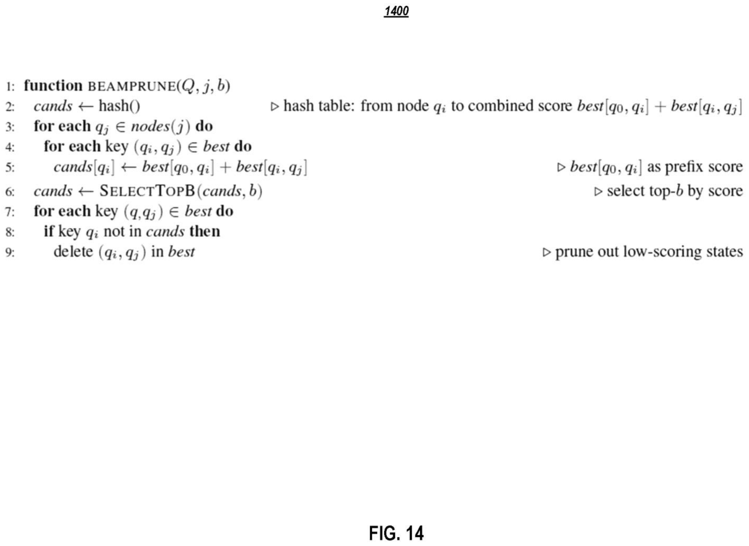

FIG. 12 gives the pseudocode of a LinearDesign methodology for the Nussinov model based on left-to-right dynamic programming and beam pruning, according to embodiments of the present disclosure. FIG. 9 and FIGs.13-14 respectively depict exemplary pseudocodes for Update function, Backtrace function (named as "BACKTRACE2" in FIG. 13 to differentiate from the BACKTRACE function listed in FIG. 10) and Beamprune function in a LinearDesign methodology, according to embodiments of the present disclosure. One shall understand that the pseudocode may be created using a computer language other than the language shown in FIG. 9 and FIGs.13-14.

### 5. Embodiments of Implementation on Turner model

In one or more embodiments, a left-to-right dynamic methodology with beam pruning is used on a Turner nearest neighbor free energy model. In one or more embodiments, thermodynamic parameters following Vienna RNAfold (Ronny Lorenz, et al., ViennaRNA package 2.0. Algorithms for Molecular Biology, 6(1):1, 2011, which is incorporated by reference in its entirety) are implemented, except for the dangling ends and special hairpins. Dangling ends refer to stabilizing interactions for multiloops and external loops, which require knowledge of the nucleotide sequence outside of the state ($q_i$, $q_j$). Though it may be integrated in LinearDesign, the implementation may become more involved. Special hairpins are hairpin loop sequences of length 3, 4, or 6 unpaired nucleotides with folding free energies stored in lookup tables, rather than estimated from features like other sequences. Special hairpins may be also integrated in LinearDesign with some preprocessing. In one or more embodiment, both dangling end and special hairpin stabilities may be included in one or more embodiments of LinearDesign.

### 6. Embodiments of MFE and codon adaptation index (CAI) joint optimization

Since CAI is also important for mRNA functional half-life, MFE and CAI may be jointly optimized. In one or more embodiments, an additive regularization term, e.g., CAI, is added in the objective function:

$$\mathbf{r}^*(\mathbf{p}) = \underset{\mathbf{r}\in\mathbf{RNA}(\mathbf{p})}{\mathbf{argmin}}(\mathbf{MFE}(\mathbf{r}) - (m+1)\lambda\log\mathbf{CAI}(\mathbf{r}))$$

$$= \underset{\mathbf{r}\in\mathbf{RNA}(\mathbf{p})}{\mathbf{argmin}}\left(\mathbf{MFE}(\mathbf{r}) - (m+1)\lambda\log(\prod_{i=1}^{m+1}w_i(\mathbf{r}))^{\frac{1}{m+1}}\right)$$

$$= \underset{\mathbf{r}\in\mathbf{RNA}(\mathbf{p})}{\mathbf{argmin}}\left(\mathbf{MFE}(\mathbf{r}) - \lambda\sum_{i=1}^{m+1}\log w_i(\mathbf{r})\right) \qquad (11)$$

where $m$ is the source sequence (e.g., a protein sequence) length, $\log w_i(r)$ is the measurement of deviation from the optimal codon (0 is optimal) for the i-th amino acid (given an mRNA candidate), $\sum_{i=1}^{m+1}\log w_i(\mathbf{r})$ is an overall cost (a sum of $\log w_i(r)$) for a target sequence r, and $\lambda$ is a hyperparameter that balances MFE and CAI.

In one or more embodiments, this equation may be integrated into a LinearDesign dynamic process, i.e., each DFA graph's edge may have a cost such that the combined cost of traversing a local path (choosing a codon) equals $\log w_i$. In one or more embodiments, each edge cost is the "best" of the paths (i.e., codons) that uses this edge.

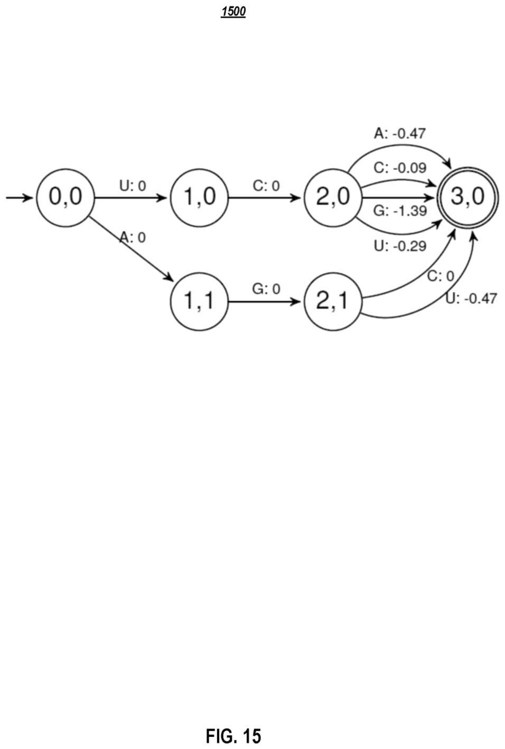

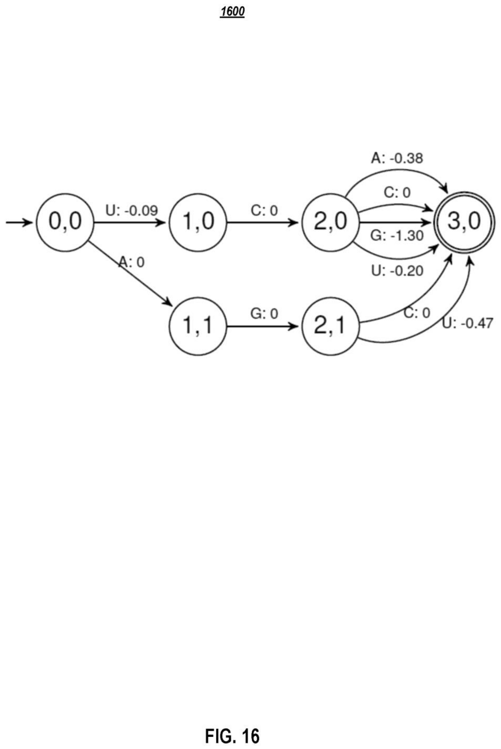

FIGs. 15 and 16 use amino acid serine as an example, showing how to integrating CAI as an edge cost in a DFA graph. In one or more embodiments, the CAI for each edge (e.g., a codon) is referred as a ratio of the frequency of the edge to the highest frequency of all edges corresponding to the source target unit (e.g., all codons translated into an amino acid in the protein sequence). A serine has 6 corresponding codons, which are listed with their CAI in Table 1 below. Each codon has a corresponding path in the DFA graph (see FIG. 15). For example, codon UCU has a frequency of 0.18, which means the codon UCG accounts for 18% of all codons in human translated into the amino acid serine. The best codon AGC has a frequency of 0.24. Therefore, the CAI for codon UCU is 0.18/0.24 = 0.75. The edge costs in "UCU path" are 0, 0, log(0.18/0.24) = -0.29, therefore, log $w_i$ of the full UCU path is -0.29. The best codon AGC has a log $w_i$ of 0, meaning that choosing AGC would not have a cost.

Table 1. Codon table of amino acid serine.

| Codon | freq. | CAI | log CAI |
|-------|-------|-----|---------|
| UCA | 0.15 | 0.63 | -0.47 |
| UCC | 0.22 | 0.92 | -0.09 |
| UCG | 0.06 | 0.25 | -1.39 |
| UCU | 0.18 | 0.75 | -0.29 |
| AGC | **0.24** | **1** | 0 |
| AGU | 0.15 | 0.63 | -0.47 |

In one or more embodiments, considering that LinearDesign is doing left-to-right dynamic programming with beam pruning and with lower scores are pruned at each step states, edge costs are incurred as early as possible in a path such that the states with better CAI paths are more likely to survive in each step. In FIG. 15, the weights only differ at the last edges. In FIG. 16, the edge costs are rearranged to fit better for LinearDesign. It shall be noted that different edge costs in FIG. 15 and FIG. 16 would not affect exact search.

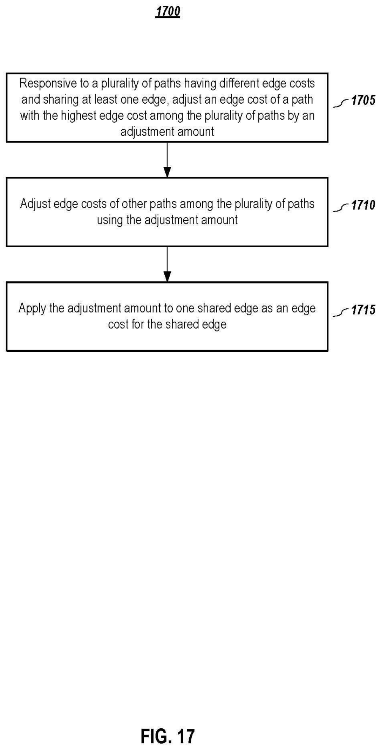

FIG. 17 depicts a process of shifting edge weight difference for edges in a DFA (or a unit DFA), according to embodiments of the present disclosure. Responsive to a plurality of paths having different edge costs (which are normally associated to a last edge of each path) and sharing at least one edge, an edge cost of a path with the highest edge cost among the plurality of paths is adjusted (1705) by an adjustment amount. In one or more embodiments, the adjustment amount is a value needed to change the highest edge cost to zero. Edge costs of other paths among the plurality of paths are adjusted (1710) using the adjustment amount. The adjustment amount is applied (1715) to one shared edge as an edge cost for the shared edge such that the overall cost for each of the paths is unchanged. In one or more embodiments, the plurality of paths have more than one share path and the earliest share path is the path that is chosen to apply the adjustment amount as the edge cost.

For the exemplary embodiment shown in FIG. 16, the edge costs in UCA, UCC, UCG, and UCU are -0.47, -0.09, -1.39, and -0.29, respectively, and differ at the last edges (between (2, 0) and (3, 0)). In order to differ the edge costs as early as possible, the highest cost (e.g., -0.09 for path UCC) among the paths (UCA, UCC, UCG, and UCU) is adjusted to 0 (such that the edge with the highest frequency has no cost), and costs of other paths are also adjusted accordingly. As a result, paths UCA, UCC, UCG, and UCU now have costs of -0.38, 0, -1.30, and -0.20, respectively, for the last edge. While the first edge (shared for all these fourth paths) has a cost adjusted from 0 into "-0.09", such that the overall cost for each of the paths (UCA, UCC, UCG, and UCU) is unchanged.

## 7. Embodiments for the top *k* best candidates

An alternative solution for finding mRNA candidates with both stable secondary structure and high CAI is to provide the top k mRNA candidates with the lowest MFE, and post-process them by features such as CAI. Although this is not as principled as the algorithm described in subsection B.6, this solution is easier to implement, and is more flexible in the sense that users may be free to add other customized filters.

In one or more embodiments, an efficient methodology is presented to find suboptimal candidates in a sorted order. During a dynamic programming process (forward-phase), instead of just saving the single best prestate as the backpointer for each state, alternative prestates that all transit to this state are stored. Then in a backtrace process (backforward-phase), starting from the last state, the second best is queried. The answer is one of the two cases: (1) the second best is from another prestate $S_1$; or (2) the second best is from the same prestate $S_0$. In the former case the second best may be found by backtracing the best path going through prestate $S_1$. While in the latter case, querying is implemented for the second best from the prestate $S_0$. Recursively, solutions, as many as needed, may be computed and obtained.

In one or more embodiments, the LinearDesign may be innovative to output suboptimal results in mRNA design. Some previous algorithms explored searching suboptimal secondary structure for RNA folding. Some found diverse suboptimal secondary structures, and some found all secondary structures in a given free energy gap. Differences between LinearDesign embodiments and these previous efforts include: (1) LinearDesign embodiments are for mRNA design, which is quite different from RNA folding; and (2) in LinearDesign embodiments, all top *k* best candidates may be output in a sorted order.

In one or more embodiments, with combination of the k-best algorithm and linearization, LinearDesign is able to quickly design a large set of mRNA candidates, which may provide a set of alternative designs for follow up with wet lab experiments.

### 8. Embodiments of less secondary structure at 5'-end leader region

Some studies have shown that protein translation level will drop if the 5'-end leader region has more secondary structure. In one or more embodiments, considering this practical issue, LinearDesign may be used to design an open reading frame (ORF) with an absence of base pairing in the 5'-end leader region by utilizing a simple strategy.

In one or more embodiments, instead of designing the most stable sequence for the whole coding region, the 5'-end leader region (e.g., the first 15 *nt)* is left unchanged from the wildtype, since the wildtype usually has less structure in this region. LinearDesign may then be used for the rest of the coding region. Because the designed region is composed of strong base pairs (generally maximizing GC content), it is unlikely for a global structure change when concatenating with the wildtype 5'-end head region, which is often depleted of GC content under observation. Refolding using secondary structure prediction tools for the concatenated sequence, its corresponding secondary structure may be obtained and it is observed that the first 15 nucleotides are unpaired.

In one or more embodiments, if a wildtype sequence is not available, all possible sequences may be alternatively enumerated in the 5'-end leader region. Because each amino acid has 3 codons on average and the start codon is fixed, the enumeration space of the first 15 *nt* in the 5'-end region is small ($3^4 = 81$), which makes the enumeration feasible.

One skilled in the art shall understand the aforementioned embodiments for LinearDesign may be used in individually, in combination or sub-combination, or in combination with one or more additional approaches for desirable functionalities.

### C. Some Results

It shall be noted that these experiments and results are provided by way of illustration and were performed under specific conditions using a specific embodiment or embodiments; accordingly, neither these experiments nor their results shall be used to limit the scope of the disclosure of the current patent document.

### 1. Efficiency and scalability

To estimate the run-time complexity of LinearDesign, 100 protein sequences from database Uniprot (UniProt Consertium. Uniprot: a hub for protein information. Nucleic Acids Research, 42:D204-D12, 2005, incorporated by reference) following CDSfold, with length from 78 to 2,828 *nt* (not including the stop codon) were used. It was found that there are three sequences whose lengths reported in CDSfold do not match the ones currently in Uniprot, so these sequences were removed, resulting in a dataset with 97 diverse protein sequences.

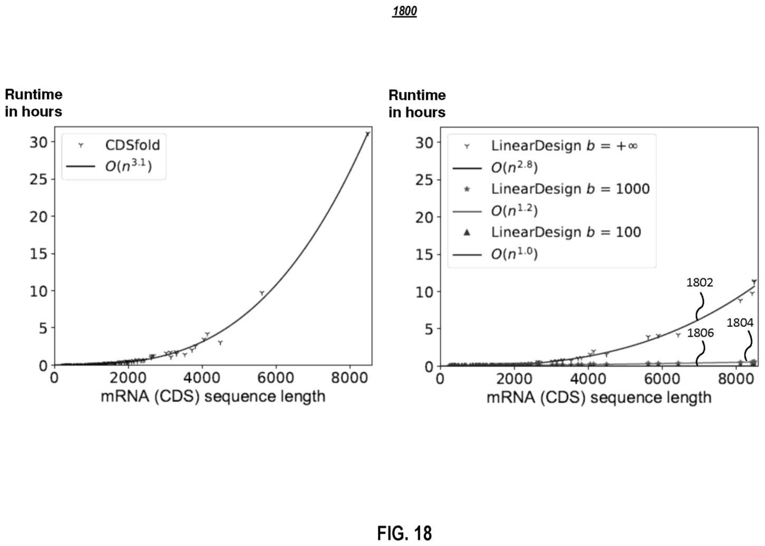

In one or more scenario, LinearDesign results were compared in exact (infinite beam size) and approximate modes (beam size *b* = 1,000 and b = 100). Runtime reported in CDSfold was used directly used to compare the computational complexity. It shall be noted that that CDSfold results and LinearDesign results were run in different machines. LinearDesign is run on a machine with 2 Intel Xeon E5-2660 v3 CPUs (2.60 GHz), while CDSfold is run on the Chimera cluster system at AIST, which was reported in the paper as 176 Intel Xeon E5550 CPUs (2.53 GHz). FIG. 18 shows runtime comparison between CDSfold and LinearDesign on Uniprot dataset. It may be observed that CDSfold, as shown in the left plot of FIG. 18, has an estimated runtime complexity of $O(n^{3.1})$, while LinearDesign (exact mode b = +∞) runs in a complexity of $O(n^{2.8})$ 1802. Both CDSfold and LinearDesign (b = +∞) have nearly cubic runtime, but LinearDesign (b = +∞) is under the exact $O(n^3)$ because the "jump" trick was used as in LinearFold, i.e., jump to the next possible nucleotide $nuc_j$ that can pair with $nuc_i$ (with the help of preprocessing), instead of checking all positions one by one. In terms of the time cost, CDSfold takes 31 hours for the longest sequence in the dataset (8,484 *nt),* while LinearDesign (b = +∞) takes 11 hours. If applying beam pruning, the runtime of LinearDesign reduces to linear complexity as expected. With beam size *b* = 1,000, the runtime is $O(n^{1.2})$ 1804, and further reduces to $O(n^{1.0})$ 1806 with b = 100. LinearDesign finishes the longest sequence design in 35 minutes with b = 1, 000, and in only 2 minutes with b = 100, which is 330× speed up compared with the LinearDesign exact search.

2. Search quality of linear-time approximation

Since the LinearDesign algorithm is significantly faster than its exact counterpart, it is envisioned that the LinearDesign algorithm may be used for long sequences. To ensure the quality of the approximation used in LinearDesign (with beam pruning mode), energy gap between the exact (b = +∞) and approximate algorithm (b = 1,000) is compared. For this analysis, the same dataset in above subsection C.1 was used.

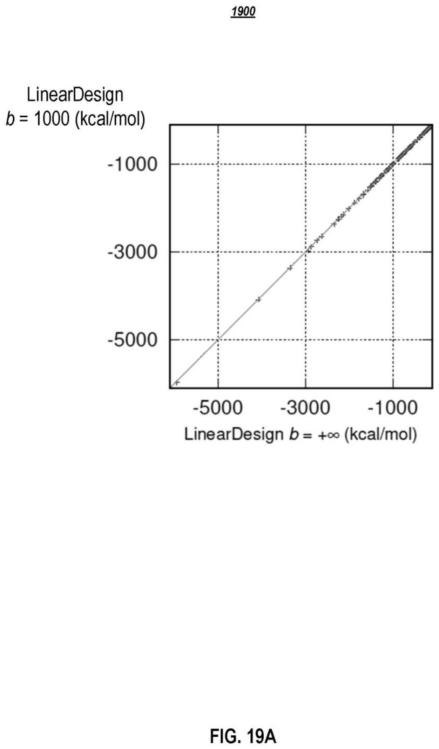

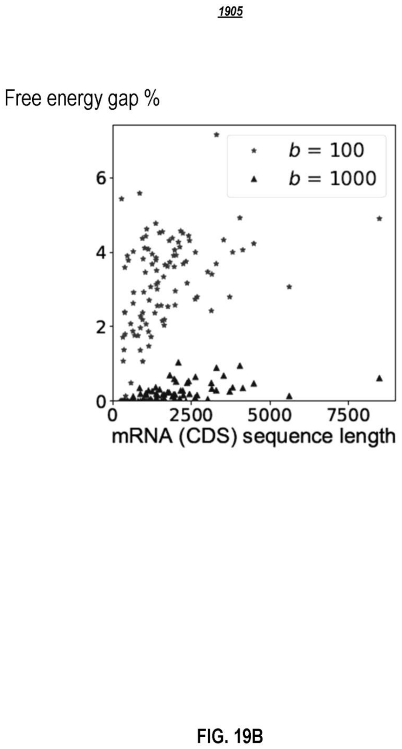

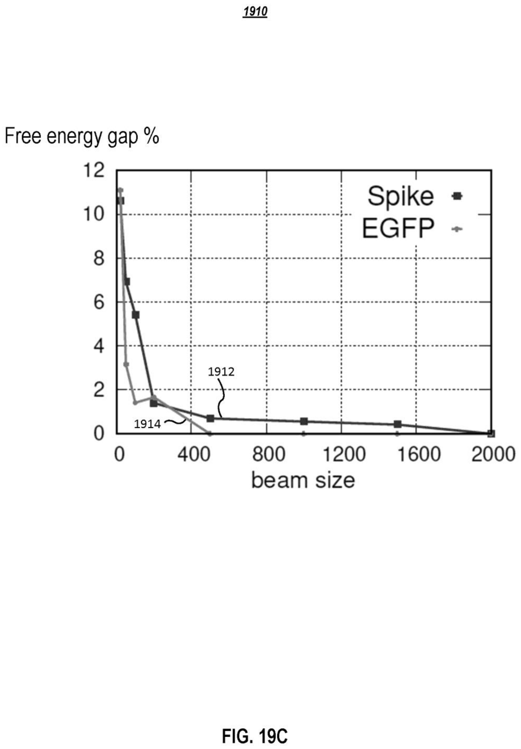

FIGs. 19A, 19B, and 19C represent the folding free energy differences between the exact search and approximation. Fig. 19A compares the free energy changes of the mRNA sequences designed with exact search and with the $b$ = 1,000 approximation. The x-axis is the free energy of exact search, while the y-axis corresponds to the free energy of approximation. It may be seen that all plots are on or close to the diagonal, which confirms that the folding free energy differences are 0 or small. Fig. 19B shows the trend of free energy differences increases linearly with mRNA sequence length. The y-axis is the percentage of free energy change gap, which is the free energy change gap ($\Delta\Delta G^0$) divided by the total free energy change of the MFE structure ($\Delta G^0$). The percentage of free energy change gap is small for all sequences in the dataset. For b = 1, 000, all sequences have gaps within 1%. Even for the longest sequence (8,484 *nt),* the gap is 0.8%. For b = 100, most gaps are within 5%, and the largest gap is 7%. Percentage of free energy change gap against beam size was also reviewed for two specific protein sequences, the spike protein of SARS-CoV-2 and EGFP (GenBank KM042177.1), as shown in Fig. 19C. The curve 1912 shows the result of the spike protein. Starting with a small beam size, $b$ = 20, the gap is 10.6%. With increasing beam size, the gap shrinks quickly to less than 6% at $b$ = 100. Further increasing b up to 500, the gap drops to 1%. With a beam size of $b$ = 2, 000 the gap drops to 0, which indicates that the approximate result is the same as the exact search. The EGFP result 1914 has the same shape as the spike protein, but the gap drops faster and down to less than 2% at $b$ = 100. This is because EGFP is shorter (239 amino acids), thus the approximation is close to the exact result even with smaller beam size. At $b$ = 200, the gap increases 0.25% compared with $b$ = 100. This happens because more states are kept when enlarging the beam size, among which states with higher scores at length j survive. Their extension to longer lengths (like offspring) beat states and fill the beams, but all these states become worse at the end, resulting a small drop of the search quality. The fluctuation happens in this sequence, but the jump is small and the gap quickly decreases to 0 at $b$ = 400 and above.

### 3. Example for the coding mRNA of the spike protein

The spike protein of SARS-CoV-2, which has 1,273 amino acid residues, is a target of mRNA vaccines. Therefore, the spike protein (GenBank QHD43416.1) of SARS-CoV-2 was used as an example for LinearDesign, and designed sequences were compared with the wildtype sequence and random generated sequences.

The mRNA sequence from a reference genome (GenBank MN908947.3) of SARSCoV-2 is used as the wildtype sequence, which contains 3,822 nucleotides (including the stop codon). Additionally, two different strategies are used to generate random sequences (5,000 sequences for each strategy) as other baselines. One of the two strategies, named "pure random", is to randomly (with equal probabilities) choose a codon for each amino acid in the spike protein, and form an mRNA sequence by concatenation. The other strategy, called "codon-biased random", is to choose a codon the probability proportional to its usage frequency. LinearDesign (in both exact mode and approximation mode) is run to evaluate the best sequences which may be achieved. Since previous studies show that both the folding free energy change of mRNA secondary structure and codon optimality influence the vaccine effectiveness, a two-dimensional comparison, MFE and CAI, is implemented between mRNA sequences.

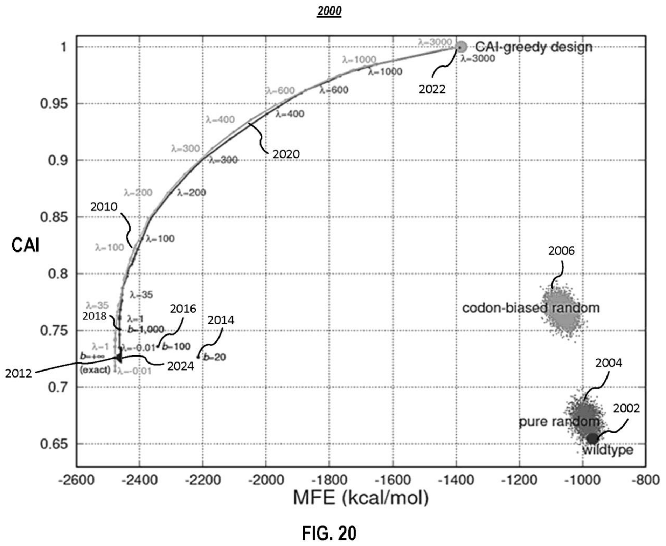

FIG. 20 depicts two-dimensional comparisons (MFE and CAI) between wildtype mRNA sequence 2002, random sequences (cloud 2004 and cloud 2006), and designed sequences (spots 2012, 2014, 2016, and 2018, curve 2010, curve 2020) on the spike protein of SARS-CoV-2. The wildtype sequence 2002 folds into a structure with the minimum free energy change of - 967.80 kcal/mol, and has a low CAI of 0.655. Most "pure random" sequences, denoted in cloud 2004, have similar free energy changes (-987.90 kcal/mol on average) as the wildtype, but with higher CAI (0.671 on average). This may be because SARS-CoV-2 just recently infected human cells and does not have enough mutations to optimize for human codons. Compared with the wildtype and "pure random" sequences, "codon-biased random" sequences, denoted in cloud 2006, have much higher CAI (0.768 on average), and slight improvement on MFE (-1063.23 kcal/mol on average). It is also noticed that both "pure random" and "codon-biased random" sequences are packed in cloud-shaped small regions. This is because the search space of possible mRNAs is extremely huge and most of the random sequences have similar MFE and CAI.

As shown in FIG. 20, designed sequences include three parts: (1) the sequence with the lowest MFE in exact mode and in approximation mode (b = 1, 000, b = 100 and b = 20, respectively), which are optimized by MFE only (shown in dark spots and labeled by beam size); (2) sequences that are jointly optimized by MFE and CAI (showed in curve 2010 for exact mode and curve 2020 for b = 1, 000); and (3) top 10, 000, 000 best sequences (dark cloud 2024 next to the b = +∞ spot 2012). FIG. 20 also shows CAI-greedy design result as a point 2022.

On the left (with much lower MFE) LinearDesign designed sequences were plotted. The plots 2012, 2014, 2016, and 2018 are optimized by MFE only. The leftmost one 2012 is the sequence designed in exact search mode, which has the lowest MFE of -2,477.70 kcal/mol and a CAI of 0.726. The MFE gap between best designed sequence using LinearDesign and the wildtype, as well as random sequences, is large (more than 1,300 kcal/mol). With only 0.56% MFE loss from the exact search sequence, the designed sequence with beam size b = 1, 000 achieves an MFE of -2,463.8 kcal/mol and a higher CAI of 0.751. Compared to the exact mode, which takes 1 hour for designing the sequence, the approximation with b = 1, 000 only takes 11 minutes, resulting in a 5.5× speed-up. For b = 100 and b = 20, the MFE are still lower than -2,200 kcal/mol, with CAI both at around 0.735 and 0.725, respectively. It may be seen from FIG. 20 that the designed sequences, for both exact search sequence and approximate search sequences, are much better than random ones and the wildtype in terms of MFE.

FIG. 20 shows the top 10, 000, 000 suboptimal sequences for exact mode (the dark cloud 2024 on the right of exact design sequence). The MFE of the sequences are very close to the optimal one, and the free energy gaps are within 20 kcal/mol. Some of the sequences have higher CAI, e.g., some have the CAI higher than 0.730. This shows that the k best algorithm may be used to select sequences with low MFE and relative higher CAI as vaccine candidates

Results of MFE and CAI joint optimization are also shown in FIG. 20. Curve 2010 is joint optimization design using exact mode. Each point on the curve is with a different $\lambda$, which balances the importance of MFE and CAI. It may be seen that the curve is on the top-left of the figure, indicating that the sequences on the curve have both stable secondary structures and higher CAI. In fact, this curve is the accessible boundary of all possible sequences, i.e., no sequences can achieve the region beyond (to the top-left) the curve. The points on the curve 2010 are good candidates for an mRNA vaccine. For example, the point with $\lambda$ = 100, has the free energy change of -2,414.6 kcal/mol and CAI of 0.823, which is only 2.5% away from the optimal MFE sequence but with 0.097 increase in CAI. It is observed that with $\lambda$ > 100, the sequences on the curve have better CAI than codon-biased random sequences. Shifting right from the curve 2010 with a small margin, curve 2020 is the results of joint optimization using b = 1,000. This curve shows that the approximation quality is good with b = 1,000. A CAI-greedy sequence, which greedily chooses the best codon for each amino acid, is designed as a special sequence with CAI = 1. It may be seen that the two curves 2010 and 2020 both point to the CAI-greedy design, confirming that the designed sequences achieve better CAI but sacrifice MFE with increasing $\lambda$, and reach CAI-greedy design with a large $\lambda$ (e.g., $\lambda$ = 3, 000).

It shall be noted that the MFE of the wildtype, the CAI-greedy design and random sequences are calculated by Vienna RNAfold with "-d0" (disable stabilizing interactions for multi-loops and external loops), to make fair comparisons with the designed sequences. All the sequences can be refolded using RNAfold without "-d0", by which the points will shift to the left.

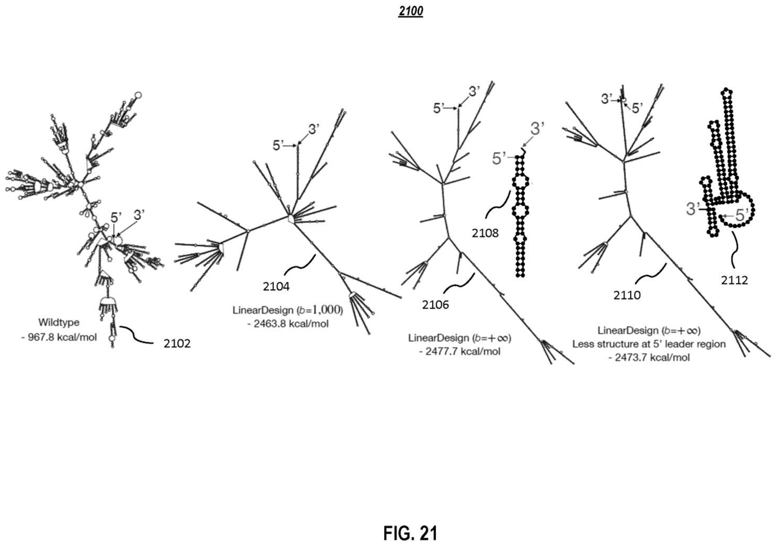

FIG. 21 shows the secondary structures of the wildtype sequences, designed sequences with b = 1,000 and b = +∞, as well as designed sequences with less structures at the 5'-end leader region. It may be seen that the secondary structures of the wildtype (Structure 2102) have a large number of loops, and designed sequences (Structure 2104 and Structure 2106) have longer helices and fewer loops, which makes the structure more stable. The designed sequence with b = 1, 000 (Structure 2104) has similar free energy changes as the one with b = +∞ (Structure 2106), but it has a multi-loop in the middle.

Additionally, the effectiveness of strategy for less structure design at the 5'-end leader region was reviewed. Structure 2110 is the whole secondary structure of the designed sequence with the goal of leaving the 5' end unpaired (the first 15 nucleotides kept identical to the wildtype and the remaining nucleotides designed by LinearDesign), and the 5'-end is zoomed in, as shown in Structure 2112. As a comparison, the 5'-end of designed sequence without constraint is also zoomed in as shown in Structure 2108. This demonstrates that the strategy may keep the 5' end unstructured, whereas designing the complete sequence results in base pairing at the 5' end.

## D. Some Discussions

The mRNA design problem is of utmost importance, especially for mRNA vaccines during the current COVID-19 pandemic. In the present disclosure, this problem is reduced into a classical problem in formal language theory and computational linguistics, namely the intersection of a CFG (encoding the energy model) with a DFA (encoding the mRNA search space). This reduction provides a natural $O(n^3)$-time CKY-style bottom-up algorithm, where n is the mRNA sequence length, but this algorithm might still be too slow for long proteins such as the spike protein of SARS-CoV-2, a promising candidate for an mRNA vaccine. In one or more embodiments, a left-to-right algorithm, LinearDesign, is developed with employment of beam search to reduce the runtime to $O(n)$, with the cost of exact search. LinearDesign is orders of magnitude faster than exact search (i.e., $b = +\infty$) and suffers only a small loss in folding free energy. For example, for this spike protein, LinearDesign may finish in 11 minutes while exact search takes 1 hour, and the free energy difference is only 0.6%. Two algorithms are also presented for incorporating codon optimality (CAI) into the consideration, one using k-best algorithms to compute suboptimal sequences and one directly integrating CAI into dynamic programming. Embodiments of the present patent document may provide efficient computational tools to speed up and improve mRNA vaccine development.

Although one or more embodiments of the present disclosure are for mRNA design, the LinearDesign may be used for other vaccine study and antibody development. One or more embodiments of the LinearDesign may be applicable, individually or in combination, for antibody sequence design.

## E. Computing System Embodiments

In one or more embodiments, aspects of the present patent document may be directed to, may include, or may be implemented on one or more information handling systems (or computing systems). An information handling system/-computing system may include any instrumentality or aggregate of instrumentalities operable to compute, calculate, determine, classify, process, transmit, receive, retrieve, originate, route, switch, store, display, communicate, manifest, detect, record, reproduce, handle, or utilize any form of information, intelligence, or data. For example, a computing system may be or may include a personal computer (e.g., laptop), tablet computer, mobile device (e.g., personal digital assistant (PDA), smart phone, phablet, tablet, etc.), smart watch, server (e.g., blade server or rack server), a network storage device, camera, or any other suitable device and may vary in size, shape, performance, functionality, and price. The computing system may include random access memory (RAM), one or more processing resources such as a central processing unit (CPU) or hardware or software control logic, read only memory (ROM), and/or other types of memory. Additional components of the computing system may include one or more disk drives, one or more network ports for communicating with external devices as well as various input and output (I/O) devices, such as a keyboard, mouse, stylus, touchscreen and/or video display. The computing system may also include one or more buses operable to transmit communications between the various hardware components.

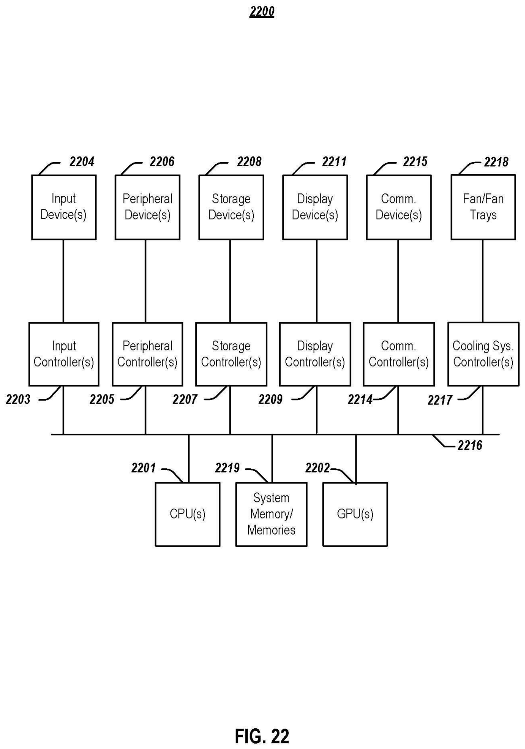

FIG. 22 depicts a simplified block diagram of an information handling system (or computing system), according to embodiments of the present disclosure. It will be understood that the functionalities shown for system 2200 may operate to support various embodiments of a computing system-although it shall be understood that a computing system may be differently configured and include different components, including having fewer or more components as depicted in FIG. 22.

As illustrated in FIG. 22, the computing system 2200 includes one or more central processing units (CPU) 2201 that provides computing resources and controls the computer. CPU 2201 may be implemented with a microprocessor or the like, and may also include one or more graphics processing units (GPU) 2202 and/or a floating-point coprocessor for mathematical computations. In one or more embodiments, one or more GPUs 2202 may be incorporated within the display controller 2209, such as part of a graphics card or cards. Thy system 2200 may also include a system memory 2219, which may comprise RAM, ROM, or both.

A number of controllers and peripheral devices may also be provided, as shown in FIG. 22. An input controller 2203 represents an interface to various input device(s) 2204, such as a keyboard, mouse, touchscreen, and/or stylus. The computing system 2200 may also include a storage controller 2207 for interfacing with one or more storage devices 2208 each of which includes a storage medium such as magnetic tape or disk, or an optical medium that might be used to record programs of instructions for operating systems, utilities, and applications, which may include embodiments of programs that implement various aspects of the present disclosure. Storage device(s) 2208 may also be used to store processed data or data to be processed in accordance with the disclosure. The system 2200 may also include a display controller 2209 for providing an interface to a display device 2211, which may be a cathode ray tube (CRT) display, a thin film transistor (TFT) display, organic light-emitting diode, electroluminescent panel, plasma panel, or any other type of display. The computing system 2200 may also include one or more peripheral controllers or interfaces 2205 for one or more peripherals 2206. Examples of peripherals may include one or more printers, scanners, input devices, output devices, sensors, and the like. A communications controller 2214 may interface with one or more communication devices 2215, which enables the system 2200 to connect to remote devices through any of a variety of networks including the Internet, a cloud resource (e.g., an Ethernet cloud, a Fiber Channel over Ethernet (FCoE)/Data Center Bridging (DCB) cloud, etc.), a local area network (LAN), a wide area network (WAN), a storage area network (SAN) or through any suitable electromagnetic carrier signals including infrared signals. As shown in the depicted embodiment, the computing system 2200 comprises one or more fans or fan trays 2218 and a cooling subsystem controller or controllers 2217 that monitors thermal temperature(s) of the system 2200 (or components thereof) and operates the fans/fan trays 2218 to help regulate the temperature.

In the illustrated system, all major system components may connect to a bus 2216, which may represent more than one physical bus. However, various system components may or may not be in physical proximity to one another. For example, input data and/or output data may be remotely transmitted from one physical location to another. In addition, programs that implement various aspects of the disclosure may be accessed from a remote location (e.g., a server) over a network. Such data and/or programs may be conveyed through any of a variety of machine-readable medium including, for example: magnetic media such as hard disks, floppy disks, and magnetic tape; optical media such as CD-ROMs and holographic devices; magneto-optical media; and hardware devices that are specially configured to store or to store and execute program code, such as application specific integrated circuits (ASICs), programmable logic devices (PLDs), flash memory devices, other non-volatile memory (NVM) devices (such as 3D XPoint-based devices), and ROM and RAM devices.

Aspects of the present disclosure may be encoded upon one or more non-transitory computer-readable media with instructions for one or more processors or processing units to cause steps to be performed. It shall be noted that the one or more non-transitory computer-readable media shall include volatile and/or non-volatile memory. It shall be noted that alternative implementations are possible, including a hardware implementation or a software/hardware implementation. Hardware-implemented functions may be realized using ASIC(s), programmable arrays, digital signal processing circuitry, or the like. Accordingly, the "means" terms in any claims are intended to cover both software and hardware implementations. Similarly, the term "computer-readable medium or media" as used herein includes software and/or hardware having a program of instructions embodied thereon, or a combination thereof. With these implementation alternatives in mind, it is to be understood that the figures and accompanying description provide the functional information one skilled in the art would require to write program code (i.e., software) and/or to fabricate circuits (i.e., hardware) to perform the processing required.

It shall be noted that embodiments of the present disclosure may further relate to computer products with a non-transitory, tangible computer-readable medium that have computer code thereon for performing various computer-implemented operations. The media and computer code may be those specially designed and constructed for the purposes of the present disclosure, or they may be of the kind known or available to those having skill in the relevant arts. Examples of tangible computer-readable media include, for example: magnetic media such as hard disks, floppy disks, and magnetic tape; optical media such as CD-ROMs and holographic devices; magneto-optical media; and hardware devices that are specially configured to store or to store and execute program code, such as application specific integrated circuits (ASICs), programmable logic devices (PLDs), flash memory devices, other non-volatile memory (NVM) devices (such as 3D XPoint-based devices), and ROM and RAM devices. Examples of computer code include machine code, such as produced by a compiler, and files containing higher level code that are executed by a computer using an interpreter. Embodiments of the present disclosure may be implemented in whole or in part as machine-executable instructions that may be in program modules that are executed by a processing device. Examples of program modules include libraries, programs, routines, objects, components, and data structures. In distributed computing environments, program modules may be physically located in settings that are local, remote, or both.

One skilled in the art will recognize no computing system or programming language is critical to the practice of the present disclosure. One skilled in the art will also recognize that a number of the elements described above may be physically and/or functionally separated into modules and/or sub-modules or combined together.

It will be appreciated to those skilled in the art that the preceding examples and embodiments are exemplary and not limiting to the scope of the present disclosure. It is intended that all permutations, enhancements, equivalents, combinations, and improvements thereto that are apparent to those skilled in the art upon a reading of the specification and a study of the drawings are included within the true spirit and scope of the present disclosure. It shall also be noted that elements of any claims may be arranged differently including having multiple dependencies, configurations, and combinations.

**Claims**

1. A computer-implemented method for sequence design comprising:

receiving a source sequence comprising multiple source sequence units (305; 705);
building a unit deterministic finite automaton (DFA) for each of the multiple source sequence units, each unit DFA comprises multiple nodes including a start node and an end node, each DFA has one or more paths between the start node and the end node, each path comprises multiple edges with each edge coupled between two adjacent nodes (310; 710);
concatenating at least unit DFAs for the multiple source sequence units into a single DFA representing candidate target sequences that translate into the source sequence (315);
intersecting a context free grammar (CFG) on the single DFA for an intersected CFG (605), wherein the CFG is a Stochastic CFG (SCFG);
defining each nonterminal and start symbol in the intersected CFG (610);
defining one or more rules in the intersected CFG (615); and
searching in the intersected CFG for a desired target sequence having a sequence structure that minimizes an objective function, wherein the desired target sequence is used as an antibody sequence;
wherein the source sequence is a protein sequence comprising multiple amino acids, the candidate target sequences are messenger ribonucleic acid (mRNA) sequences that translate into the protein sequence, each mRNA sequence comprises multiple codons;

wherein the objective function comprises a minimum free energy (MFE) of a target sequence and an additive regularization term, the additive regularization term is an overall cost traversing a full path of the target sequence; wherein the overall cost is a sum of combined edge costs for all codons on the target sequence, each combined edge cost is associated to a codon on the target sequence; wherein the combined edge cost for the codon is obtained by:

for an amino acid related to the codon, obtaining frequencies for all codons that translate into the amino acid; taking a relative ratio of the frequency of the codon to the highest frequency among all codons as a codon adaption index (CAI) for the codon; and obtaining the combined edge cost for the codon by implementing a log operation for the CAI;

wherein searching in the intersected CFG for a desired target sequence having a sequence structure that minimizes an objective function comprising:

initializing a first hash table to store a best score for each state between two nodes in the single DFA and a second hash table to store a best backpointer for the each state (715); initializing a singleton for a state of each adjacent node pair (720); when the searching in the intersected CFG goes to a current node,

going through one or more pairing rules for state update for each state between two nodes before the current node when node number difference between the two nodes is larger than a predetermined value (725); going through one or more bifurcation rules for state and backpointer updates each state between two nodes before the current node (730); keeping a predetermined number of states and pruning out the rest states; and

back-tracing at least the desired target sequence after the searching in the intersected CFG goes to a last node in the single DFA (735).

2. The computer-implemented method of claim 1 wherein back-tracing at least the desired target sequence after the searching in the intersected CFG goes to a last node in the single DFA further comprising: back-tracing a plurality of top target sequences in a sorted order.

3. A non-transitory computer-readable medium or media comprising one or more sequences of instructions which, when executed by at least one processor, causes steps for sequence design comprising:

receiving a source sequence comprising multiple source sequence units (1105); building a unit deterministic finite automaton (DFA) for each of the multiple source sequence units (1110), each unit DFA comprises multiple nodes including a start node and an end node, each DFA has one or more paths between the start node and the end node, each path comprises multiple edges with each edge coupled between two adjacent nodes; concatenating at least unit DFAs for the multiple source sequence units into a single DFA representing candidate target sequences that translate into the source sequence, each target sequence comprising multiple target sequence units respectively translating into the multiple source sequence units; intersecting a context free grammar (CFG) on the single DFA for an intersected CFG, wherein the CFG is a Stochastic CFG (SCFG); searching in the intersected CFG for a desired target sequence having a sequence structure that minimizes an objective function, wherein the desired target sequence is used as an antibody sequence; wherein the objective function comprises a minimum free energy (MFE) of a target sequence and an additive regularization term, the additive regularization term is an overall cost traversing a full path of the target sequence; wherein the overall cost is a sum of combined edge costs for all target sequence units on the target sequence, each target sequence unit on the target sequence has a combined edge cost; wherein the source sequence is a protein sequence, the source sequence units are amino acids, the target sequence is an mRNA sequence, the multiple target sequence units are codons; wherein the combined edge cost for a codon is obtained by:

obtaining frequencies for all codons that translate into an amino acid; taking a relative ratio of the frequency of the codon to the highest frequency among all codons as a codon adaption index (CAI) for the codon; and

obtaining the combined edge cost for the codon by implementing a log operation for the CAI;

wherein searching in the intersected CFG for a desired target sequence having a sequence structure that minimizes an objective function comprising:

initializing a first hash table to store a best score for each state between two nodes in the single DFA and a second hash table to store a best backpointer for the each state (715);
initializing a singleton for a state of each adjacent node pair (720);
when the searching in the intersected CFG goes to a current node,

going through one or more pairing rules for state update for each state between two nodes before the current node when node number difference between the two nodes is larger than a predetermined value (725);
going through one or more bifurcation rules for state and backpointer updates each state between two nodes before the current node (730);
keeping a predetermined number of states and pruning out the rest states; and

back-tracing at least the desired target sequence after the searching in the intersected CFG goes to a last node in the single DFA (735).

4. The non-transitory computer-readable medium or media of claim 3, wherein the steps for sequence design further comprises:

responsive to multiple paths in the unit DFA having different combined edge cost and sharing at least one edge, adjusting a combined edge cost for a path with the highest combined edge cost among the multiple paths by an adjustment amount (1705);
adjusting combined edge costs of other paths among the multiple paths using the adjustment amount (1710);
applying the adjustment amount to one shared edge as an edge cost for the shared edge such that the combined edge cost for each of the multiple paths remains unchanged (1715).

FIG. 1

FIG. 2

_300_

Receive a source sequence comprising multiple source sequence units — *305*

Build a unit DFA for each of the multiple source sequence units, each unit DFA has a start node and an end node with one or more paths between the start node and the end node — *310*

Concatenate unit DFAs for the multiple source sequence units into a single DFA — *315*

**FIG. 3**

FIG. 4

FIG. 5

_**600**_

Intersect a CFG (*G*) on a single DFA (*D*) corresponding to an source sequence for an intersected CFG (*G'*) — 605

Define each nonterminal and start symbol in the intersected CFG — 610

Define one or more rules in the intersected CFG — 615

**FIG. 6**

<u>*700*</u>

```
┌─────────────────────────────────────────────────────┐
│ Receive a source sequence comprising multiple source │ ⌐ 705
│                    sequence units                    │
└─────────────────────────────────────────────────────┘
                          │
                          ▼
┌─────────────────────────────────────────────────────┐
│         Build a single DFA for the source sequence   │ ⌐ 710
└─────────────────────────────────────────────────────┘
                          │
                          ▼
┌─────────────────────────────────────────────────────┐
│ Initialize two hash tables, one table (best) to store│ ⌐ 715
│ the best score for each state, and another table     │
│ (back) to store the best backpointer for each state  │
└─────────────────────────────────────────────────────┘
                          │
                          ▼
┌─────────────────────────────────────────────────────┐
│ Initialize base case (signleton) for a state of each │ ⌐ 720
│                    adjacent node pair                │
└─────────────────────────────────────────────────────┘
                          │
                          ▼
┌─────────────────────────────────────────────────────┐
│ For each state ... go through one or more pairing    │ ⌐ 725
│ rules for state update                               │
└─────────────────────────────────────────────────────┘
                          │
                          ▼
┌─────────────────────────────────────────────────────┐
│ For each state ... go through one or more bifurcation│ ⌐ 730
│ rules for state update                               │
└─────────────────────────────────────────────────────┘
                          │
                          ▼
┌─────────────────────────────────────────────────────┐
│ Back-trace the best target sequence with stored      │ ⌐ 735
│ backpointers after the hash tables are filled out    │
│ bottom-up                                            │
└─────────────────────────────────────────────────────┘
```

- Box 725: For each state $(q_i, q_j)$ between two nodes $q_i$ and $q_j$ where the number difference between $i$ and $j$ is larger than a predetermined value, go through one or more pairing rules for state update
- Box 730: For each state $(q_i, q_j)$, go through one or more bifurcation rules for state update

**FIG. 7**

*800*

**function** BOTTOMUPDESIGN(p)

    $n \leftarrow 3 \cdot |\text{p}| + 1$     ▷ mRNA length; +1 for the stop codon

    $D \leftarrow D(x_1) \circ D(x_2) \circ \dots \circ D(\text{stop})$     ▷ build protein DFA

    $best \leftarrow \text{hash}()$     ▷ hash table: from $[q_i, q_j]$ to score

    $back \leftarrow \text{hash}()$     ▷ hash table: from $[q_i, q_j]$ to backpointer

    **for** $i = 0 \dots (n-1)$ **do**

        **for each** $q_i \in \text{nodes}(D, i)$ **do**

            **for each** $(q_{i+1}, nuc_i) \in \text{out\_edges}(D, q_i)$ **do**

                $best[q_i, q_{i+1}] \leftarrow 0$     ▷ singleton: $N \xrightarrow{0} \text{A} \mid \text{C} \mid \text{G} \mid \text{U}$

                $back[q_i, q_{i+1}] \leftarrow nuc_i$

    **for** $l = 2 \dots n$ **do**

        **for** $i = 0 \dots (n-l)$ **do**

        $j \leftarrow i + l$

        **for each** $q_i \in \text{nodes}(D, i)$ **do**

            **for each** $q_j \in \text{nodes}(D, j)$ **do**

                **if** $j - i > 4$ **then**     ▷ pairing (no sharp turn)

                    **for each** $(q_{i+1}, nuc_i) \in \text{out\_edges}(D, q_i)$ **do**

                        **for each** $(q_{j-1}, nuc_{j-1}) \in \text{in\_edges}(D, q_j)$ **do**

                        **if** $\text{match}(nuc_i, nuc_{j-1})$ **then**

                            $score \leftarrow best[q_{i+1}, q_{j-1}] - 1$   ▷ $S \xrightarrow{-1} \text{A} \, S \, \text{U} \mid \dots$

                            UPDATE$(q_i, q_j, score, (nuc_i, q_{i+1}, q_{j-1}, nuc_{j-1}))$

                  **for** $k = (i+1) \dots (j-1)$ **do**     ▷ bifurcation

                    **for each** $q_k \in \text{nodes}(D, k)$ **do**

                        $score \leftarrow best[q_i, q_k] + best[q_k, q_j]$     ▷ $S \xrightarrow{0} S \, S$

                        UPDATE$(q_i, q_j, score, q_k)$

    **return** $best[q_0, q_n]$, BACKTRACE$(q_0, q_n)$

**FIG. 8**

EP 4 682 890 A2

_900_

```
1:  function UPDATE(q_i, q_j, score, backpointer)
2:     if score < best[q_i, q_j] then                    ▷ minimizing weight
3:        best[q_i, q_j] ← score
4:        back[q_i, q_j] ← backpointer
```

**FIG. 9**

```
1: function BACKTRACE(q_i, q_j)
2:    backpointer ← back[q_i, q_j]
3:    if type(backpointer) is string then                          ▷ singleton
4:       nuc_i ← backpointer
5:       return nuc_i, " . "
6:    if length(backpointer) = 4 then                              ▷ pairing: S →⁻¹ A S U | ...
7:       nuc_i, q_{i+1}, q_{j-1}, nuc_{j-1} ← backpointer
8:       seq, struct ← BACKTRACE(q_{i+1}, q_{j-1})
9:       return nuc_i + seq + nuc_{j-1}, " ( " + struct + " ) "
10:   else                                                          ▷ bifurcation: S →⁰ S S
11:      q_k ← backpointer
12:      seq_1, struct_1 ← BACKTRACE(q_i, q_k)
13:      seq_2, struct_2 ← BACKTRACE(q_k, q_j)
14:      return seq_1 + seq_2, struct_1 + struct_2
```

EP 4 682 890 A2

FIG. 10

_1100_

Receive a source sequence comprising multiple source sequence units — *1105*

Build a single DFA for the source sequence — *1110*

Initialize two hash tables, one table (*best*) to store the best score for each state, and another table (*back)* to store the best backpointer for each state — *1115*

Initialize base case (signleton) for a state of each adjacent node pair — *1120*

For each state between two nodes before a current node, go through one or more pairing rules for state update when node number difference between the two nodes is larger than a predetermined value — *1125*

For each state between two nodes before the current node, go through one or more bifurcation rules for state and backpointer updates — *1130*

For the current node, keep a predetermined number of states and prune out the rest states — *1135*

Back-trace the best target sequence after the process goes to the last node — *1140*

**FIG. 11**

*1200*

```
1:  function LINEARDESIGN(p, b)                              ▷ b is beam size
2:      n ← 3 · |p| + 1              ▷ mRNA length; +1 for the stop codon
3:      D ← D(x₁) ∘ D(x₂) ∘ ... ∘ D(stop)        ▷ build protein DFA
4:      best ← hash()                      ▷ hash table: from [qᵢ, qⱼ] to score
5:      back ← hash()            ▷ hash table: from [qᵢ, qⱼ] to backpointer
6:      for i = 0 ... (n − 1) do
7:          for each qᵢ ∈ nodes(D, i) do
8:              for each (qᵢ₊₁, nucᵢ) ∈ out_edges(D, qᵢ) do
9:                  best[qᵢ, qᵢ₊₁] ← 0          ▷ singleton: N →⁰ A | C | G | U
10:                 back[qᵢ, qᵢ₊₁] ← nucᵢ
11:     for j = 1 ... n do
12:         for each qⱼ₋₁ ∈ nodes(D, j − 1) do
13:             for each qᵢ such that [qᵢ, qⱼ₋₁] ∈ best do
14:                 for each (qⱼ, nucⱼ₋₁) ∈ out_edges(D, qⱼ₋₁) do
15:                     backpointer ← (qⱼ₋₁, nucⱼ₋₁)
16:                     UPDATE(qᵢ, qⱼ, best[qᵢ, qⱼ₋₁], backpointer) ▷ S →⁰ S N
17:                 if j − (i − 1) > 4 then
18:                     for each (qᵢ₋₁, nucᵢ₋₁) ∈ in_edges(D, qᵢ) do
19:                         if match (nucᵢ₋₁, nucⱼ₋₁) then  ▷ S →⁻¹ S A S U | ....
20:                             for each qₖ such that [qₖ, qᵢ₋₁] ∈ best do
21:                                 score ← best[qₖ, qᵢ₋₁] + best[qᵢ, qⱼ₋₁] − 1
22:                                 backpointer ← (qᵢ₋₁, nucᵢ₋₁, qᵢ, qⱼ₋₁, nucⱼ₋₁)
23:                                 UPDATE(qₖ, qⱼ, score, backpointer)
24:             BEAMPRUNE(best, j, b)  ▷ choose top-b among all (qᵢ, qⱼ)'s
25:     return best[q₀, qₙ], BACKTRACE2(q₀, qₙ)
```

**FIG. 12**

```
1: function BACKTRACE2(q_i, q_j)
6:    if length(backpointer) = 5 then                                          ▷ pairing: S →⁻¹ S A S U | ...
7:       q_{k-1}, nuc_{i-1}, q_k, q_{j-1}, nuc_{j-1} ← backpointer
8:       seq_1, struct_1 ← BACKTRACE2(q_i, q_{k-1})
9:       seq_2, struct_2 ← BACKTRACE2(q_k, q_{j-1})
10:      return seq_1 + nuc_{i-1} + seq_2 + nuc_{j-1}, struct_1 + " (" + struct_2 + ") "
11:   else                                                                      ▷ unpaired: S →⁰ S N
12:      q_{j-1}, nuc_{j-1} ← backpointer
13:      seq, struct ← BACKTRACE2(q_i, q_{j-1})
14:      return seq + nuc_{j-1}, struct + " . "
```

**FIG. 13**

EP 4 682 890 A2

```
1: function BEAMPRUNE(Q, j, b)
2:    cands ← hash()                          ▷ hash table: from node qᵢ to combined score best[q₀, qᵢ] + best[qᵢ, qⱼ]
3:    for each qⱼ ∈ nodes(j) do
4:      for each key (qᵢ, qⱼ) ∈ best do
5:        cands[qᵢ] ← best[q₀, qᵢ] + best[qᵢ, qⱼ]                        ▷ best[q₀, qᵢ] as prefix score
6:    cands ← SELECTTOPB(cands, b)                                       ▷ select top-b by score
7:    for each key (q, qⱼ) ∈ best do
8:      if key qᵢ not in cands then
9:        delete (qᵢ, qⱼ) in best                                       ▷ prune out low-scoring states
```

$$\text{cands}[q_i] \leftarrow best[q_0, q_i] + best[q_i, q_j]$$

FIG. 14

FIG. 15

_1600_

FIG. 16

_1700_

Responsive to a plurality of paths having different edge costs and sharing at least one edge, adjust an edge cost of a path with the highest edge cost among the plurality of paths by an adjustment amount — 1705

Adjust edge costs of other paths among the plurality of paths using the adjustment amount — 1710

Apply the adjustment amount to one shared edge as an edge cost for the shared edge — 1715

**FIG. 17**

FIG. 18

EP 4 682 890 A2

FIG. 19A

_1905_

Free energy gap %

FIG. 19B

_1910_

FIG. 19C

FIG. 20

EP 4 682 890 A2

**FIG. 21**

_2200_

**FIG. 22**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GORO TERAI et al.** CDSfold: an algorithm for designing a proteincoding sequence with the most stable secondary structure. *Bioinformatics*, 2016, vol. 32 (6), 828-834

- Uniprot: a hub for protein information. *Nucleic Acids Research*, 2005, vol. 42, D204-D12